# EUROPEAN PATENT SPECIFICATION

(11) **EP 1 084 278 B1**
(45) Date of publication and mention of the grant of the patent: **19.10.2005**
(21) Application number: 99955506.3
(22) Date of filing: 05.06.1999
(51) Int. Cl.: C12Q 1/68

(54) **DETECTION OF DNA, RNA AND PROTEINS**
ERKENNUNG VON DNS, RNS UND PROTEINEN
DETECTION D'ADN, D'ARN ET DE PROTEINES

(30) Priority: 08.06.1998 US 93532
(43) Date of publication of application: 21.03.2001
(73) Proprietor: ACGT Medico Inc., Toronto, Ontario M5G 1Z2 (CA)
(72) Inventor: CHEN, Hai Xing, Toronto, Ontario M5G 2A1 (CA)
(74) Representative: Müller-Boré & Partner Patentanwälte
(86) International application number: PCT/IB1999/001037
(87) International publication number: WO 1999/064628

(56) References cited:
- EP-A- 0 387 696
- EP-A- 0 605 828
- EP-A- 0 780 479
- EP-A- 0 846 776
- GB-A- 2 283 569
- GB-A- 2 324 370

## Description

The present invention relates to a column and method for the detection of test materials in small concentrations, especially the detection of pathogen indicators. In particular it relates to the detection of DNA, RNA and proteins in serum.

Historically, the diagnosis of diseases has depended upon clinical manifestations. However, new techniques of detecting diseases have been developed with the advent of nucleic acid and monoclonal antibody detection methods. The detection of nucleic acid has been used for diseases associated with abnormal gene products, such as anemia, Huntington's disease and certain thalassemia mutations. In addition, the detection of nucleic acid has been used for bacterial and viral diseases, such as Human Immunodeficiency Virus (HIV). Moreover, monoclonal antibody detection methods have gained acceptance for the identification and differentiating of certain diseases such as cancers.

As appreciated by those skilled in the art, the detection of a pathogen indicator has applicability to the detection of certain diseases associated with abnormal genes, certain diseases associated with the presence of an identifiable nucleic acid sequence and certain diseases associated with the immune system. The pathogen indicator described herein includes DNA, RNA, antibody, antigen, and other proteins.

Known manual pathogen indicator detection methods in research and clinical laboratories tend to have low accuracy, low sensitivity and are subject to human error, both in carrying out the methods and in interpreting the results. Other methods, e.g. culturing methods, are not suitable for many diseases. For example, tuberculosis has a very slow growth rate, which makes detection not easy or even not possible.

U.S. Patent 5,753,439 to Smith et. al. describes a method to detect characteristic dinucleotide and trinucleotide acid sequences, to determine target sequences and to screen for genetic defects and disorders associated with the sequences. The assays are conducted on solid surfaces allowing for multiple reactions to be conducted. However, the method does not provide a control process to provide assurances that the results are accurate and sensitive to determining if there is an error in the method.

U.S. Patent 5,824,478 to Muller describes a method wherein a sample is contacted with a detector probe and a capture probe to form a detector probe-analyte-capture probe complex which is used to detect a target analyte in a sample. The target analytes, capture probes, and detector probes can be nucleic acids and polypeptides. However, the method does not provide a control process to provide assurances that the results are accurate and sensitive to determining if there is an error in the method.

In a standard enzyme ELISA method for immunoassay, a tray with a plurality of wells, e.g. 96 wells, containing appropriate antibodies is used. One method to eliminate error in this ELISA is to use a control. One of the wells can be used as a positive control (with a positive antigen), while the remaining wells can be used for testing patient's sera. After addition of the serum samples, the wells are washed and a second antibody, which carries an enzyme, is added to the wells. After washing again, a substrate is added. The substrate and enzyme react, with a color reaction. The color yield from the reaction is associated with the presence of an antigen. The method is rife with possibilities for error. Human error can lead to some wells being washed twice or not at all, having reagents added twice or not at all, or wells being inadvertently contaminated with extraneous materials. For example, over washing tends to flush all the components and create a false negative result, while an incomplete wash will provide detection from non-binding materials and yield false positive results. The control well can give no assurance that the results from any other well is indicative of the presence or otherwise of the pathogen indicator under investigation. Additionally, color differences from well to well give additional uncertainties with respect to interpretation of the results.

EP-A-0846776 published after the date of priority of the present application teaches an assay device for isolating a plurality of different analytes from a sample. The device comprises a capillary tube containing a linear array of binding elements that are each linked to distinct binding factors, to which a corresponding specific component binds. The binding elements are stacked directly on top of one another to form a packed column.

Most of the previous tests are demanding of time, skill and concentration. So much so, that in many jurisdictions the number of tests that can be conducted by one technician is limited by regulation. This serves to raise the cost of testing, as it is so labour dependent.

For all the above reasons, and more, a new method, column and a kit for detecting a pathogen indicator is desirable, which is accurate, reproducible, and is sensitive to determining if there is an error in the method.

The present invention provides a method for detecting the presence of a test material in a test sample. The method comprises the steps of: (a) introducing a test sample and a control material into a test column, wherein the column has at least two snares, one of said snares having thereon a control capture material; at least one of said snares having thereon a target capture material specific to a corresponding test material in the test sample for which the detection is being sought, so that the control capture material will bind with the control material to form a bound control material; and the target capture material will bind with the corresponding test material to form a bound material; (b) washing the test column to remove any materials which have not been bound to the capture materials; and (c) detecting the presence of bound materials on each of the snares. The method can further comprise adding a label material for each of the bound materials to form labeled bound materials and then detecting the presence of the labeled bound materials.

In another embodiment, the present invention provides a method for detecting the presence of a DNA sequence in a test sample. The method comprises the steps of: (a) denaturing a test sample to form a single strand target DNA sequence for which detection is being sought; (b) introducing the test sample and a first control single strand DNA sequence into a test column which has at least two snares, one of said snares having a first control single strand capture DNA sequence; at least one of said snares having thereon a target single strand capture DNA sequence specific to the corresponding target DNA sequence in the test sample; and wherein the target single strand capture DNA sequence will bind with the corresponding target DNA sequence in the test sample to form a double strand DNA sequence, and the first control single strand capture DNA sequence will bind with the first control DNA sequence to form a double strand control DNA sequence; (c) adding a wash solution to the column to remove unbound DNA; (d) adding an enzyme to the column to destroy single strand DNA; (e) adding a denaturing solution to separate the formed double strand DNA sequences, then adding a wash solution to remove denatured non-capture single strand sequences, so that the single strand capture DNA sequences re-form on each snare; (f) adding DNA probes to provide detectable labels for single strand capture DNA sequences formed in step (e); (g) adding a wash solution to the column to remove unbound DNA probes; and (h) detecting any signals from each snare. In step (b), the first single strand control DNA sequence can be added into said test sample prior to introducing the sample into the test column, or can be added into the test column separately from the test sample. Moreover, the method can further include adding a substrate which reacts with the labels to give off detectable signals.

Additionally, the method further comprises introducing a second control single strand DNA sequence into the test column; wherein the test column has a control snare thereon having a second control single strand capture DNA sequence.

In a further embodiment, the snares have more than one single strand capture DNA sequences on one single snare, and the labels are different for different single strand capture DNA sequences on one single snare so that different DNA sequences can be detected on one single snare.

In yet another embodiment, the method for detecting the presence of a DNA sequence in a test sample comprises the steps of: (a) providing a positive control single strand DNA sequence; (b) denaturing a test sample to form a single strand target DNA sequence for which detection is being sought; (c) adding the test sample and the positive control DNA sequence to a test column, wherein the column has at least two snares, one of said snares having thereon a first control single strand capture DNA sequence for binding to a portion of the positive control DNA sequence; at least one of said snares having thereon a target single strand capture DNA sequence specific to the corresponding target DNA sequence in the test sample, so that the positive control DNA sequence binds with the first control single strand capture DNA sequence wherein the bound positive control DNA sequence has a double strand portion and a single strand portion; and the target DNA sequence present in the test sample binds with the target single strand capture DNA sequence wherein the bound target DNA sequence has a double strand portion and a single strand portion; (d) adding a wash solution to the column to remove unbound DNA; (e) adding DNA probes to provide detectable labels for attachment to the single strand portion of the bound positive control DNA sequence and the single strand portion of the bound target DNA sequence formed in step (c); (f) adding a wash solution to the column to remove unbound DNA probes; and (g) detecting any signals each snare. In addition, the method can further include adding a substrate which reacts with the labels to give off detectable signals.

The positive control single strand DNA sequence is prepared from a target DNA sequence for which detection is being sought, by a process selected from the group consisting of (1) inserting a control DNA fragment into the target DNA sequence for which detection is being sought at a predetermined scission point; and (2) removing a small fragment of DNA from the target DNA sequence at a predetermined scission point.

Further more, step (c) of the method can further include adding a negative control DNA sequence to the test column; wherein the test column also has a control snare having thereon a second control single strand capture DNA sequence for binding to the negative control DNA sequence, so that the negative control DNA sequence binds with the second control single strand capture sequence to form a bound negative control DNA sequence. The negative control single strand DNA sequence is different from the target DNA sequence and different from the positive control DNA sequence.

In a further aspect, the present invention provides a method for detecting the presence of a RNA sequence in a test sample. A method for detecting the presence of a RNA sequence in a test sample comprises the steps of: (a) providing a positive control single strand DNA sequence; (b) adding a test sample and the positive control DNA sequence to a test column wherein the column has at least two snares, one of said snares having thereon a first control single strand capture DNA sequence for binding to the positive control DNA sequence; at least one of said snares thereon having a target single strand capture DNA sequence specific to the corresponding target RNA sequence in the test sample, so that the positive control DNA sequence binds with the first control capture DNA sequence to form a double strand positive control DNA sequence, and the RNA sequence present in the test sample binds with the target capture DNA sequence to form a double strand DNA/RNA complex; (c) adding a wash solution to the column to remove unbound positive control DNA and target RNA; (d) adding an enzyme to the column to destroy single strand DNA and RNA; (e) adding a denaturing solution to separate the formed double strand control DNA sequence and double strand DNA/RNA complex, then adding a wash solution to remove denatured non-capture single strand DNA and RNA sequences, so that the single strand capture DNA sequences re-form on each snare; (f) adding DNA probes to provide detectable labels for single strand capture DNA sequences formed in step (e); (g) adding a wash solution to the column to remove unbound DNA probe; and (h) detecting any signals from each snare. In addition, the method can further include adding a substrate which reacts with the labels to give off detectable signals.

In one embodiment, the positive control single strand DNA sequence is different from the target RNA sequence and the first control single strand capture DNA sequence is different from the target single strand capture DNA sequence. In addition, the DNA probes used in step (f) are different for the first control capture and the target capture sequences.

In another embodiment, the positive control single strand DNA sequence has a portion which has the same sequence to a portion of the target RNA sequence. The first control single strand capture DNA and the target single strand capture DNA have a common sequence at a portion of the capture sequences, so that a common DNA probe is used in step (f) for detection of the re-formed control and target capture sequences.

In a further embodiment, step (a) of the RNA detection method further includes providing a negative control single strand DNA sequence which is different from the target RNA sequence and different from the positive control DNA sequence. Step (b) further includes adding the negative control DNA to the test column which also has a control snare having thereon a second control single strand capture DNA sequence. The second control capture DNA sequence partially matches the negative control DNA sequence so that the negative control DNA sequence binds with the second control capture DNA sequence to form a double strand DNA sequence which also has unbound single strand portions. Step (f) DNA probes do not match re-formed partial second control single strand capture DNA sequence formed in step (e), and no binding occurs between them. Therefore, in step (h) no signal is detected from the second control snare under normal conditions.

In yet another embodiment, the second control single strand capture DNA sequence is different from the target capture DNA and the first control capture DNA sequences.

In an additional embodiment, the first control capture DNA, the second control capture DNA and the target capture DNA have a common sequence at a portion of the capture sequences. A common DNA probe is used in step (f) for detection of the re-formed control and target capture sequences.

In another aspect, the present invention provides a column for analysis of a test material, wherein the column has at least two snares, one of said snares having thereon a first control capture material for detecting the presence of a first control material, and at least one of said snares having thereon a test capture material for detecting a test material for which detection is being sought. The column can also comprise at least two chambers, each chamber having a snare, one of said chambers having a first control capture material on the snare for detecting the presence of a first control material, and at least one of said chambers having a test capture material on the snare for detecting the test material for which detection is being sought.

In a further embodiment, the chambers have a connecting means to connect different chambers in order, and the chambers are connected along the longitudinal axis of the chamber through the connecting means. Furthermore, the snares can reside on a snare tray which is in a plane transverse to a longitudinal stem. When the stem rotates, the snares will be conveyed to the sample station, reagent stations, and detection stations.

Additionally, the column further has a chamber or a snare having a second control material on the snare for detecting the presence of a second control material.

In a further aspect, the present invention provides a kit. The kit comprises (a) a column for analysis of a test material, wherein the column has at least two snares, one of said snares having thereon a first control capture material for detecting the presence of a first control material, and at least one of said snares having thereon a capture material for detecting a test material for which detection is being sought; (b) reagents for detecting the presence of the test materials.
Figures 2A and 2B show a reagent delivery station for delivering a reagent to a column.
Figure 3 shows a detector for detecting a signal from a column.
Figure 4 shows a column made with four chambers.
Figures 5A, 5B and 5C show a process of one embodiment of the present invention, schematically.
Figure 6 shows read-outs from analyses of six samples using the method of the present invention, wherein each sample is analyzed with two controls. In the figure, A is the signal of a positive control, C is the signal of a negative control, and B is the signal of a test sample.
Figure 7 illustrates a method for detecting a DNA sequence in a sample.
Figure 8 illustrates another method for detecting a DNA sequence in a sample.
Figure 9A and 9B illustrate two modes of a method for detecting a RNA sequence in a sample.
Figures 10A and 10B show a column and a sample dispenser prior to use, and in use, respectively. Figure 10C is a partial cross-sectional view of the column and sample holder of Figures 10A and 10B just prior to connection of the sample holder and the puncturer.
Figure 11 shows a snare tray ensemble comprising a longitudinal stem and a plurality of snare tray which has a plurality of snares on each tray.

The test column can have different shape and configurations. In general, the column has at least two snares, one of the snares having thereon a first control capture material for detecting the presence of a first control material, and the other snare having thereon a capture material for detecting a test material for which detection is being sought. The column can also have at least two chambers, each chamber having a snare, one of the chambers having a first control capture material on the snare for detecting the presence of a first control material, and the other chamber having a capture material on the snare for detecting a test material for which detection is being sought. Detail descriptions and examples of the test columns are given hereinafter.

A sample can also be added into the test column by a syringe pump or manually.

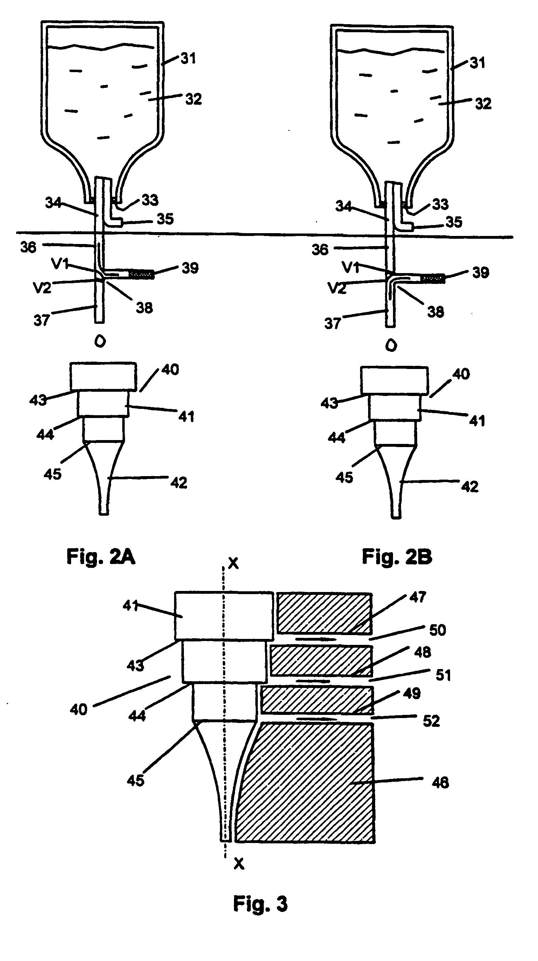

Figures 2A and 2B show one type of reagent station and one type of column. At the reagent station there is a reagent bottle 31 with reagent 32 therein. The reagent bottle 31 has a rubber septum 33 through which a double-hollow needle 34 may penetrate. The double-hollow needle 34 has one tube 35 which is connected to an air supply (not shown). A second tube 36 is connected to a delivery tube 37 by a double valve junction 38. Also connected to double valve junction 38 is a measuring syringe 39. Second tube 36 and delivery tube 37 may be a single tube with a double valve junction therein. Vatves V1 and V2 in double valve junction 38 allow reagent 32 to be drawn into measuring syringe 39 and then be expelled from measuring syringe 39 through delivery tube 37. Column 40 is situated just below delivery tube 37 so that reagent may be transferred, e.g. by gravity, to column 40. Alternatively there may be a liquid-tight connection between delivery tube 37 and column 40. The reagents may be urged through the columns by means of pressure from the top or vacuum attached to the discharge tube.

In another aspect, the present invention also provides a column for analysis of a test material in a sample. The test material herein includes, but not limited to, DNA, RNA, PNA, antibody, antigen, protein and a material that specifically binds to DNA, RNA and proteins. Preferably, the test material is a pathogen indicator, such as DNA, RNA and antigen. The column has at least two snares, one of said snares having thereon a first control capture material for detecting the presence of a first control material, and the other of said snares having thereon a test capture material for detecting a test material for which detection is being sought. The column can also have at least two chambers, at least one of the chambers containing a snare thereon having a first control capture material, and at least one of the chambers containing a snare having thereon a test capture material for detecting a test material for which detection is being sought. Each chamber has a connecting means to connect the chambers in order.

The term of test material used here means the material in a test sample for which detection is being sought. It is also referred to as target material, target protein, target DNA, target RNA, target antigen depending on the specific application. The term of test capture material means the capture material that specifically binds with a test material for which detection is being sought. The test capture material is also referred to as target capture material, target capture protein and target capture DNA depending on its specific application.

In addition to a snare having thereon a first control material, a column can have a plurality of snares each having thereon a different capture material for detecting more than one test material in a test sample. Optionally, a column can further have a snare having thereon a second control capture material for detecting the presence of a second control material. Moreover, a column can have an additional chamber with a snare without a capture material thereon. This snare can be used for detecting background signals of the detection method.

The capture materials for detecting the control materials and the test material for which detection is being sought include, but not limited to, single strand DNA sequence, antibody, antigen and protein. The selection of appropriate capture material depends on the specific detection being sought.

Alternatively, a snare tray can also be used for the method of detection. The snare tray has a plurality of snare locations in a plane transverse to a longitudinal stem. A detection station has a plurality of detectors which can be used for detecting the presence of control or test materials at the snare locations.

Figure 3 shows one type of column. Column 40 comprises a column casing 41 and a discharge tube 42. Column casing 41 houses three snares 43, 44 and 45, which are spaced apart from one another. The snares are spaced apart along longitudinal axis X-X of column 40. Although three snares are shown in Figures 2A and 2B, there should be at least two snares in a column. At least one of the snares is used for detecting a control material, and at least one of the snares is used for detecting the presence or otherwise of a material in a test sample. Snares 43-45 may be made from any suitable material for attaching a capture material as will be explained in more detail hereinafter. Typically the snares are made from a material with high surface area, e.g. sintered glass, sintered plastic, glass fibre, beads, chips, granules, and membrane. When membrane is used, a solid support may be required. One example of a snare is a layer of fine latex particles thereon having capture antibodies attached covalently, wherein the latex particles spread out on a porous sintered glass plate. The snares may sometimes be referred herein as glass frits or a fibre chips. The column casing adjacent to the snares are preferably light transparent, for better detection of chemiluminescent or other chemical reaction.

It is preferable that the snares be in a particular order, so that there may be positive identification of any reaction at a particular snare position. Clearly, it is important that detection of any reaction be identified with a particular capture material. For example, it is important that detection of any reaction with a control capture material, e.g. albumin, be positively identified with that control capture material and not with any other capture material, e.g. tuberculosis capture material. For this reason it is preferable that there is means to ensure that the order of the snares and their associated capture materials follows a predetermined order.

The column 40 may take any convenient shape, cylindrical, square, rectangular, or cylindrical with one flat side. In the Figures 2A, 2B, 3, 5A to 5C, column 40 is a step-shaped tube. Such a shape makes it necessary to make snares 43, 44 and 45 to be of different diameters. As will be described in more detail hereinafter, each of the snares may have a different capture material attached thereto. As described in general above, it may be important that a snare with a first capture material always be placed in the top position 43. Making snare 43 in a larger diameter than the other snares ensures that snare 43 cannot be placed in the position reserved for snare 44 or snare 45. Conversely, making a snare 45 in small diameter, with a different capture material to the capture material on snare 43, ensures that snare 45 cannot be placed in the position of snare 43 or 44. Obviously this is helpful in ensuring that the snares are correctly placed in the column. Of course, columns with correctly placed snares may be accomplished in other ways and so the stepped-tube arrangement shown in Figures 2A, 2B, 3, 5A to 5C is not essential. As will be apparent, the column may have a circular cross-section, a square cross-section or other suitable shape.

As will be described hereinafter at least one of the snares, e.g. 43 is used as a control. In some situations a second snare, e.g. 45 is also used as a second control, as will be described in reference to use of the columns and apparatus. The third snare, e.g. 44, is for testing for presence of a particular chemical from a sample in a test tube, e.g. a pathogen indicator in a patient's serum.

In Figure 2A, the upper valve V1 of double-valve junction 38 is open and the lower valve V2 is closed, to allow a measured amount of reagent to be drawn into measuring syringe 39. In Figure 2B, the upper valve V1 of double-valve junction 38 is closed and the lower valve V2 is open, to allow the measured amount of reagent in measuring syringe 39 to be expelled through delivery tube 37.

Figure 3 shows column 40 adjacent to detector means. In the embodiment shown, in order to avoid false readings, detector block 46 is shaped to accommodate the shape of column casing 41. Detector block 46 has channels 47, 48, 49 for allowing any signals emanating from snares 43, 44, 45 to pass to detectors 50, 51 52, respectively. Another advantage of the stepped column casing as shown in Figure 3 is that signals from each of the snares are prevented from filtering through to an adjacent detector channel. Although UV detectors may be used in certain instances, laser detectors are preferred.

Some reactions are chemiluminescent and detection of the chemiluminescence may be determined directly from light emanating from the snare, as implied from the positioning of channels 47-49 in Figure 3. However, sometimes it may be necessary to measure the light emanating at an angle from the top surface of each snare. Accordingly, the channels would then be angled to guide such light to an appropriate detector.

Other reactions may require a different detection system. For example, it may be necessary to have a light source for detecting certain reactions, as will be understood by those skilled in the art. In addition, columns different from those of Figure 2A may require different arrangements for the detection apparatus, as will be described hereinafter.

Although Figure 3 shows a means, for example, for minimizing cross-over of light from snare 43 to channel 48, if reaction times are in the order of milliseconds and there is a substantial time interval between reactions from adjacent snares, it may not be as critical to prevent light cross-over from one detector to another.

Signals from the detectors may be displayed in a number of ways. For example, the signals may be displayed graphically on paper or on a monitor. The signals may also be manipulated to assess the concentration of pathogen indicators on the various snares. The data from the signals may be stored electronically and then retrieved either locally or remotely. In addition to the data, software can be used to provide information concerning the tests performed and thus enhance the viewer's understanding and interpretation of the results. One of the advantages of remote access to the results is that a doctor who requests the tests may review the raw data both rapidly and directly, without requiring the assistance of and interpretation by a technician. Because there is at least one control test in each column, the doctor can immediately assess whether the tests have been done correctly and thus have a high degree of confidence in any result which shows the presence or absence of the pathogen indicator under consideration. Additionally, because the tests can be conducted rapidly, a doctor may be able to have tests performed at relatively short time intervals and soon thereafter be able to see if the concentration of pathogen indicator is increasing or decreasing.

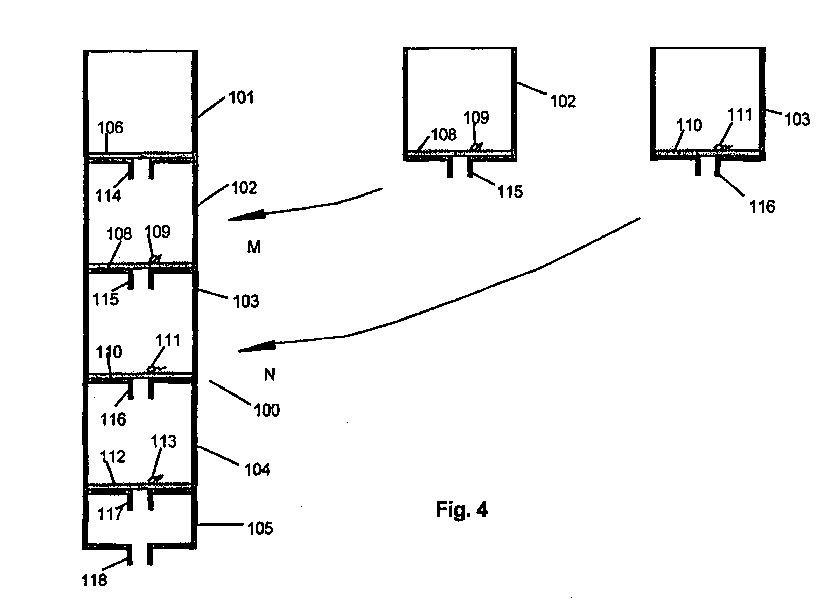

Another arrangement of the column is shown in Figure 4. Column 100 is constructed from four chambers 101, 102, 103 and 104 and a discharge chamber 105. Discharge chamber 105 has a discharge spout 118. Chambers 102 and 103 are shown to the right of completed column 100, with arrows M and N to show the placement of chambers 102 and 103 in the column. There are means to assure that chamber 102 can only be connected to the bottom of chamber 101, that chamber 103 can only be connected to the bottom of chamber 102, and that chamber 104 can only be connected to the bottom of chamber 103. One suitable example for ensuring connection among different chambers with correct order is to have embedded slots at a specific position for different chambers, or to have different numbers of slots for different chambers.

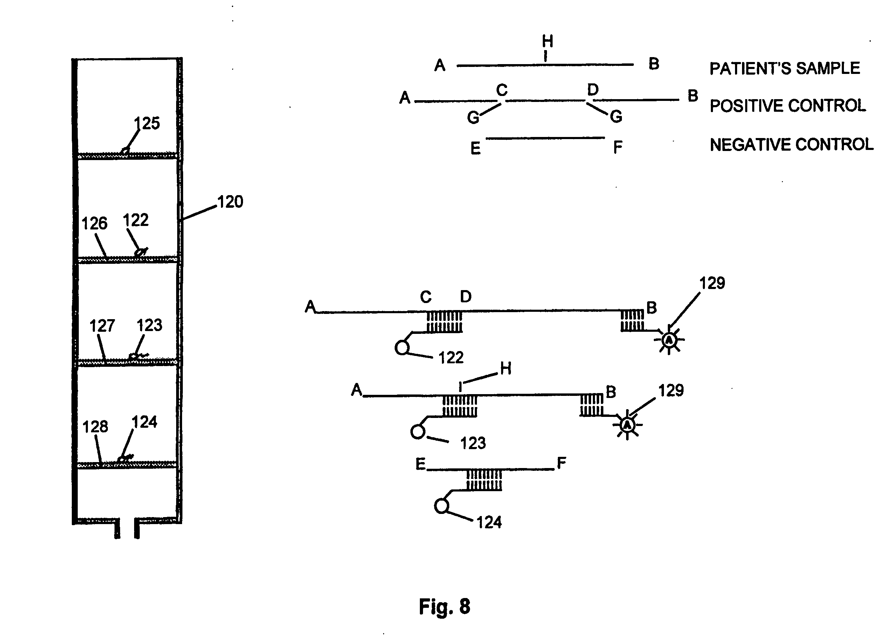

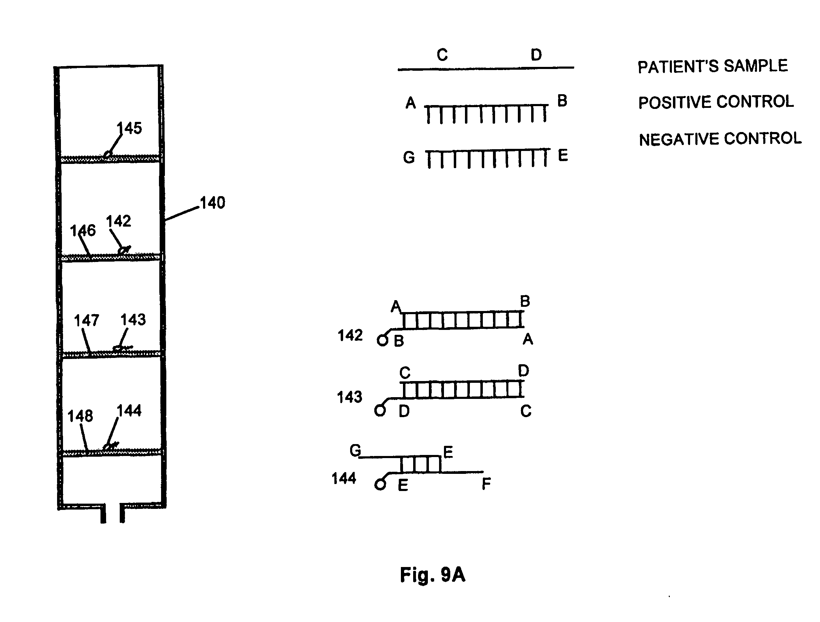

In the embodiment shown in Figure 4, chamber 101 has a drain 114, a snare 106 which has no capture material thereon. Chamber 102 has a drain 115, and a snare 108 with a first control capture material 109. Chamber 103 has a drain 116 and a snare 110 with a pathogen indicator capture material 111. Chamber 104 has a drain 117, and a snare 112, with a second control capture material 113. The significance of the four chambers with their associated capture materials will be described hereinafter, particularly in relation to a pathogen indicator detection method using DNA. The presence of drain 114, 115, 116 and 117 is optional. When the snare is made of glass frit with appropriate pore size, there is no need of a drain. Such a snare is shown in column 120 of Figure 8 and column 140 of Figure 9A. On the other hand, if a membrane is used as a snare, a solid support is needed and a drain can be preferred.

In some countries, used columns must be discarded rather than being reused, whereas in other countries reuse of columns is permitted. In situations where reuse is permitted, an automated cleaning process is preferred in order to assure that the columns are not contaminated or otherwise inoperative. In such a process, the used column is washed with a reagent that destroys everything except the capture materials on the snares. After such washing, the column is tested for the presence of unwanted materials. If there are still unwanted materials on the column, then further washing and detection sequences are carried out. If, after a number of washings, a particular column is still not clean, the column is discarded and replaced by a new column.

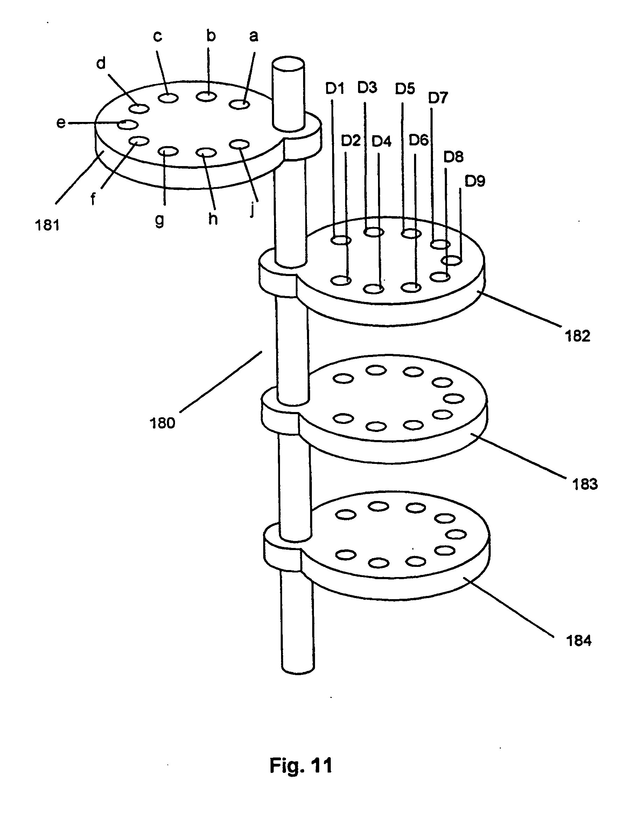

Figure 11 illustrates a snare tray which provides an alternative ensemble of snares for detecting a test material. There is a stem 180 with a plurality of snare trays 181, 182, 183 and 184. Each snare tray has a plurality of snares thereon, e.g. a, b, c, d, e, f, g, h and j on tray 181, which may be viewed as equivalent to a column. Snares a and b for example may be for detection of positive and negative control materials, leaving snares c to j for detection of seven different pathogen indicator materials. The snares may be subjected to methods of binding, labelling and detection substantially as described hereinbefore. Because the snares on any one tray are in a plane transverse to the longitudinal direction of the stem 180, detection is best performed by placing the detectors above the trays. For example, detection may be made with detectors D1 to D9. In the embodiment shown in Figure 11, it will be understood that detection of the materials on snare 181 has been completed and tray 181 has been swung out of the way of detectors D1 to D9. Detectors D1 to D9 are in position for detecting capture and labelled materials on the snares of tray 182. Thus each tray is adaptable to detection of a large number of materials, e.g. pathogen indicators, with a single tray.

Another aspect of the invention provides a method for detecting the presence of a protein in a test sample. The method comprises the steps of:
(a) adding a sample and a control material to a column wherein the column has at least two snares, one of said snares having thereon a first control capture material for binding to the control material; at least one of said snares having thereon a target capture material for binding to the target protein in the test sample for which detection is being sought, so that the control material binds with the first control capture material to form a bound control material, and target protein present binds with the test capture material;
(b) adding a wash solution to the column to remove unbound control material and unbound target protein;
(c) adding a labelling material to the column, to bind with the control material and bound target protein;
(d) adding a wash solution to the column to remove unbound labelling material; and
(e) detecting any detectable signals from the labelled and bound control material and from any labelled and bound target protein.

The method can further include adding a substrate which reacts with the labels to give off detectable signals. The control material can be added into the column separately from the test sample. Alternatively, the control material can be added into the test sample prior to addition of the sample into the test column.

The target protein in the above process includes antigen, antibody and other proteins. When the target protein is an antigen, the capture material is an antibody which specifically binds to the target antigen. The labeling material can be a primary antibody, which binds to the bound target antigen, having thereon a chemical or enzyme label. Furthermore, the above described process is not limited to protein analysis. When a test material is capable of binding to a capture material specifically, the method can be applied.

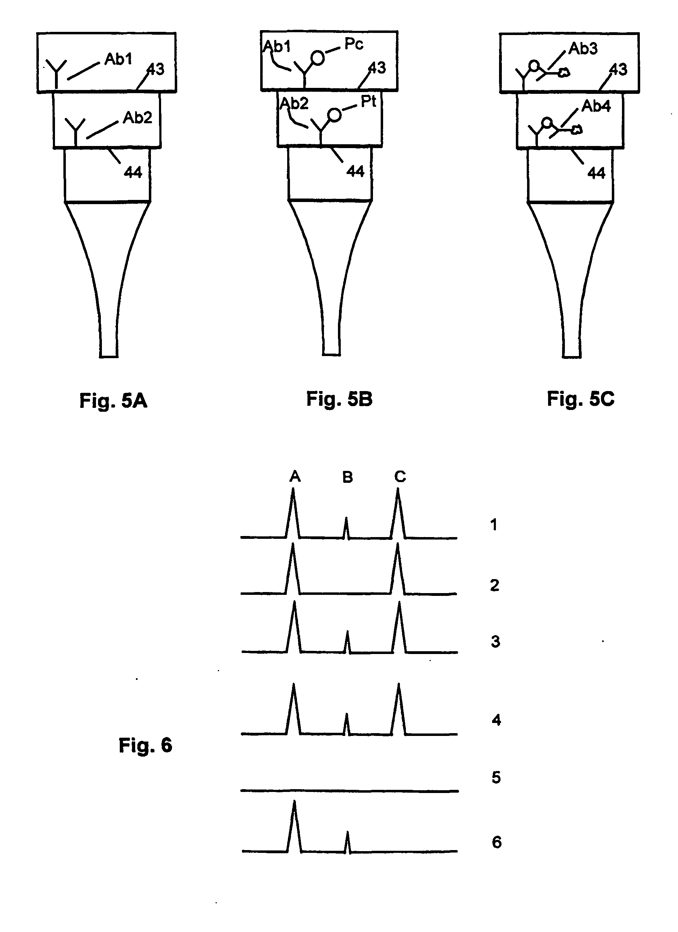

One process falling within the scope of the invention is now described with respect to a protein detection in a serum sample. Figures 5A, 5B and 5C generally show the process in simplified terms, in which there is detection of a control protein Pc, e.g. albumin, and a protein Pt whose presence is being tested for, e.g. a tuberculosis protein. The control protein Pc is present in the serum and it is not known whether the tuberculosis protein Pt is present in a patient serum sample. The control protein Pc is already inherently in the sample in a test tube or had been deliberately added into the sample beforehand. Alternatively, the control protein Pc can be added into the column separately from the patient sample, either prior to or after addition of the patient sample. Snare 43 is a sintered glass frit which has a first capture antibody Ab1 attached thereto, and snare 44 is a sintered glass frit which has a second capture antibody Ab2 attached thereto, as shown in Figure 5A. Snares 43 and 44 are in the same column. As indicated, the serum which is in the test tube contains the control protein Pc. For the purposes of this illustration, it is assumed that the protein Pt which is to be tested for, e.g. a tuberculosis protein, is present. The serum travels through snares 43 and 44. Control protein Pc binds to the first capture antibody Ab1 on snare 43, and the protein Pt binds to the second capture antibody Ab2 on snare 44, as shown in Figure 5B. At the first reagent station, a wash is administered to the column. The purpose of the wash is to remove any excess serum, including unbound control protein Pc and protein Pt.

It will be understood that transfer of serum to the column may also be automated. At the time of transfer of the serum to the column, the correlation of the identity of the serum test tube and the corresponding column are noted, either by the technician, e.g. by typing identification numbers into a computer, or by automatic methods such as bar coding of test tubes and columns.

After washing at the first reagent station, the column is then indexed to a second reagent station. At the second reagent station, a primary antibody Ab3 which will bind to the control protein Pc, is added to the column. Primary antibody Ab3 has, for example, a chemiluminescent label. After indexing to a third reagent station, a primary antibody Ab4 which will bind to the protein Pt, is added to the column, as shown in Figure 5C. Primary antibody Ab4 also has, for example, a chemiluminescent label. The column is then indexed to a fourth reagent station which is a wash station. The wash removes any excess primary antibodies Ab3 and Ab4.

The column is then indexed to a fifth reagent station which is also adjacent to a detector block. At the fifth reagent station a trigger solution is added to the column. The trigger solution reacts with the labels of the primary antibodies Ab3 and Ab4. Assuming that the reactions are chemiluminescent, there are emanations of light from each of the snares 43 and 44. The light signal from snare 43 is then detected by detector 50 and the light signal from snare 44 is detected by detector 51. Light from snare 43 indicates the presence of the control protein Pc on the snare and light from snare 44 indicates the presence of the protein Pt on the snare.

It will be appreciated from the above discussion that if there is no protein Pt in the serum, then obviously there can be no binding of such protein to capture antibody Ab2. Thus there would be nothing for primary antibody Ab4 to react with and so there would be no chemiluminescence detected by detector 51.

The advantage of using the control protein Pc is that if there is no signal detected by detector 50 after adding the trigger solution, then it indicates operation errors. If there is a signal from the control protein Pc, then the person analyzing the results can be reasonably assured that an absence of signal from snare 44 truly indicates that either no protein Pt present in the serum, or the protein Pt concentration is below the detection limit.

The control protein may be a protein that is always present in a serum sample, e.g. albumin, or a control protein may be added deliberately to the serum, or into the column directly.

It is sometimes desirable to have a second control protein (P2), which would be used to indicate whether all of the steps in the process have been carried out. For example, snare 45 may have a third capture antibody Ab5 (not shown) thereon. After addition of the serum, with the first control protein Pc and possibly the protein Pt, and washing of the column, the second control protein P2 may be added. Second control protein P2 would bind to third capture antibody Ab5. Third capture antibody Ab5 may be activated by addition of a primary antibody Ab6 which has a label, e.g. a chemiluminescent label. Now, instead of adding a reagent which only has primary antibodies Ab3 and Ab4 as described in previous paragraphs, a reagent which has primary antibodies Ab3, Ab4 and Ab6 is added to column 40. After washing and addition of a trigger solution, any reaction with primary antibody Ab6 would be detected by detector 52. It should be noted that the second control protein may be added to the column together with the first control protein instead of separately, if desired.

It will be appreciated that if there is no detection of the second control protein P2, it can be safely assumed that the primary antibodies Ab3 and Ab4 were not added to the column. Thus there is a further indication of whether the complete analytical process has been performed properly.

Although described above in relation to detection of pathogen indicators in serum samples, the above method may be adapted for detection of viruses or chemicals such as drugs, carcinogens, explosives, opiates, pollutants.

Figure 6 shows the results of a method used where there are two control snares A and C and a snare B, which is used to detect for the presence of a particular pathogen indicator. In Figure 6, six samples are tested in columns 1-6. Sample columns 1, 2, 3 and 4 show the presence of both control proteins, thus indicating that the serum is present, and that the correct primary antibodies for the first control and pathogen indicator were added. Sample columns 1, 3 and 4 show the presence of the pathogen indicator in snare B, whereas in sample 2 there is no pathogen indicator detected. Because both the control signals are positive, the operator can be reasonably assured that the lack of a signal for the pathogen indicator, in sample column 2 indicates that there is indeed no pathogen indicator present. In sample 5, neither of the control signals appear, indicating that the sample column never contained any control protein and thus lack of signal for snare B is meaningless. The negative result could be false.

In sample 6, the second control is not present. This indicates that the correct primary antibodies for detection of the first control and the pathogen indicator were not added. Thus sample 6 gives a false indication of the presence of the first control and the pathogen indicator. Detection of materials on the snares A and B has been detected, which may or may not be the first control and the pathogen indicator. Therefore, sample 6 is also meaningless.

The advantage of the present invention over prior methods is that sample 5 in prior methods would have been counted as a definite negative, i.e. it is a false negative for snare B and that sample 6 in prior methods would have been counted as a definite positive, i.e. it is a false positive for snare B.

A further aspect of the present invention provides a method for detecting the presence of a DNA sequence in a test sample. The method comprises the steps of:
(a) denaturing a test sample to form a single strand target DNA sequence for which detection is being sought;
(b) introducing the test sample and a first single strand control DNA sequence into a test column, wherein the column has at least two snares, one of said snares having thereon a first control single strand capture DNA sequence; at least one of said snares having thereon a target single strand capture DNA sequence specific to the corresponding target DNA sequence in the test sample; and wherein the target single strand capture DNA sequence will bind with the corresponding target DNA sequence in the test sample to form a double strand control DNA sequence; and the first control single strand capture DNA sequence will bind with the first control DNA sequence to form a double strand control DNA sequence;
(c) adding a wash solution to the column to remove unbound DNA;
(d) adding an enzyme to the column to destroy any single strand DNA;
(e) adding a denaturing solution to separate the formed double strand DNA sequences, then adding a wash solution to remove denatured non-capture single strand sequences, so that the single strand capture DNA sequences re-form on each snare;
(f) adding DNA probes to provide detectable labels for single strand capture DNA sequences formed in step (e);
(g) adding a wash solution to the column to remove unbound DNA probe; and
(h) detecting any signals from each snare.

The method can further include adding a substrate which reacts with the labels to give off detectable signals. The first single strand control DNA sequence can be added into the test sample prior to introducing the sample into the test column, or can be added into the test column separately from the test sample. The method further comprises introducing a second single strand control DNA sequence into the test column; wherein the test column also has a control snare having thereon a second control single strand DNA capture sequence. With the method of the present invention, one single snare can also have more than one single strand capture DNA sequences thereon. In this case, the labels are different for different single strand capture DNA sequences on one single snare so that different DNA sequences can be detected on one single snare.

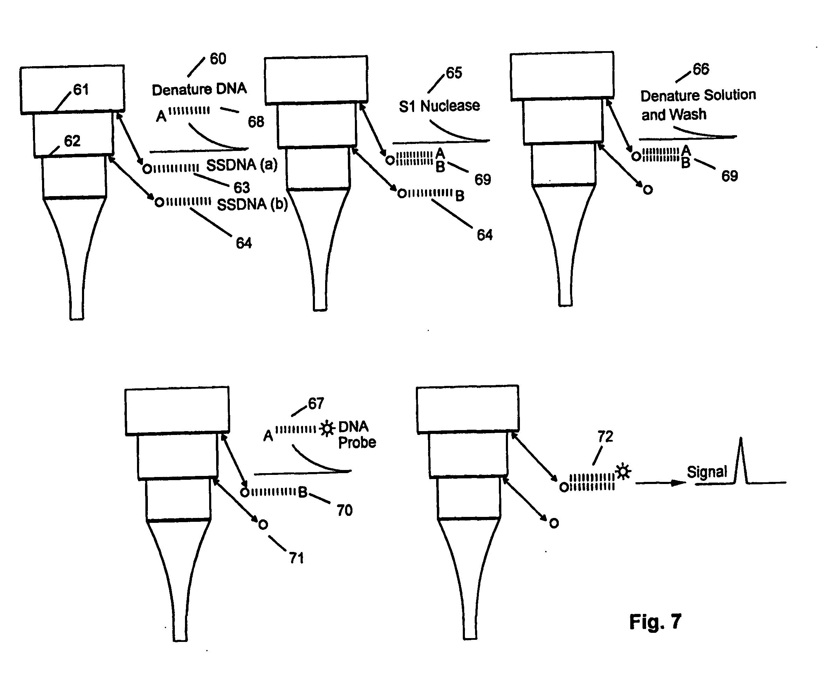

Figure 7 shows the use of the above described method for DNA analysis. In this particular embodiment it is not necessary to amplify the DNA using, for example, PCR techniques. Single strand synthetic DNA (SSDNA(a)) for the control (control single strand capture DNA sequence) and single strand DNA (SSDNA(b)) for the target pathogen indicator (target single strand capture DNA sequence) are provided in the column on snares 61 and 62, to provide the equivalent of the capture materials (shown by 63 and 64 respectively in Figure 7) discussed previously.

The patient sample is prepared so that the DNA, if present, has been isolated. The isolated target DNA as well as the control DNA are denatured into single strand DNA form and then are applied to the column (shown by 60 in Figure 7). The control DNA and the target DNA if present will bind specifically to the single strand control capture DNA sequence and the target single strand capture DNA sequence, respectively. The denatured control DNA and the target DNA in the patient sample is equivalent to the control protein and the target protein respectively in reference to Figures 5A to 5C.

The column is washed to remove non-binding DNA. An enzyme that specifically destroys single strand DNA and RNA, such as S1 nuclease 65, is then added to the column. As is known, S1 nuclease destroys only single strand DNA and RNA, not double strand DNA or RNA/DNA complex. Therefore if there has been no combination of either the SSDNA(a) or SSDNA(b), the single strand DNA will be destroyed. However, if either SSDNA(a) OR SSNDA(b) has been combined to form double strand DNA, the double strand DNA will not be destroyed. Following a washing step, a further denaturing solution 66 is added to the column. This denaturing solution separates any double strand DNA into single strand DNA. The column is again washed.

Following washing, labelled DNA probes for the control and target DNA, are added to the column. Preferably, a single labelled DNA probe 67, suitable for both control and target DNA is used. Finally, the column is washed to remove any non-binding probes. The presence of any labelled DNA probes is then detected using appropriate detection means, for example, with trigger solution and detectors.

In the process illustrated by Figure 7, the denatured sample 60 only has denatured control DNA 68 therein. There is no target pathogen indicator present. Therefore only SSDNA(a) 63 is annealed with control single strand DNA 68 to form double strand DNA 69. Because there is no denatured target DNA, SSDNA(b) remains as a single strand. Therefore, after addition of S1 nuclease, SSDNA(b) is destroyed, leaving only control double strand DNA 69. Control double strand DNA 69 is denatured with denature solution 66, leaving single strand DNA 70 (which is the same as SSDNA(a)). There is no single strand target DNA to denature (71). Finally a labelled DNA probe 67 is added, which forms a double strand control DNA 72, which may then be detected. In this instance, only the control DNA would be detected because there was no target DNA present.

In a further embodiment, the present invention provides another method for detection of DNA sequence in a test sample. The method comprises:
(a) providing a positive control single strand DNA sequence;
(b) denaturing a test sample to form a single strand target DNA sequence;
(c) adding the test sample and the positive control DNA sequence to a test column, wherein the column has at least two snares, one of said snares having thereon a first control single strand capture DNA sequence for binding to a portion of the positive control DNA sequence; at least one of said snares having thereon a target single strand capture DNA sequence specific to the corresponding target DNA sequence in the test sample, so that the positive control DNA sequence binds with the first control single strand capture DNA sequence wherein the bound the positive control DNA sequence has a double strand portion and a single strand portion; and the target DNA sequence present in the test sample binds with the target single strand capture DNA sequence wherein the bound the target DNA sequence has a double strand portion and a single strand portion;
(d) adding a wash solution to the column to remove unbound DNA;
(e) adding DNA probes to provide detectable labels for attachment to the single strand portion of the bound positive control DNA sequence and the single strand portion of the bound target DNA sequence formed in step (c);
(f) adding a wash solution to the column to remove unbound DNA probe; and
(g) detecting any signals from each snare.

The method can further include adding a substrate which reacts with the labels to give off detectable signals. The positive control single strand DNA sequence is prepared from a target DNA sequence for which detection is being sought, by a process selected from the group consisting of (1) inserting a control DNA fragment into the target DNA sequence for which detection is being sought at a predetermined scission point; and (2) removing a small fragment of DNA from the target DNA sequence at a predetermined scission point;

Moreover, the method can further include adding a negative control DNA sequence to the test column. In this case, the test column also has a control snare having thereon a second control single strand capture DNA sequence for binding to the negative control DNA sequence, so that the negative control DNA sequence binds with the second control single strand capture sequence to form a bound negative control DNA sequence. The negative control single strand DNA sequence is different from the target DNA sequence and different from the positive control DNA sequence.

The control DNA sequences can be added into the test column separately from the test sample, or can be added into the test sample prior to the addition of the test sample to the test column.

This method is exemplified in Figure 8. A target DNA sequence, for which detection is sought, is selected, e.g. tuberculosis DNA sequence. A mutation is formed by known techniques of insertion of a DNA sequence or deletion of a portion of the target DNA sequence. For example, in relation to Figure 8, such a mutated target DNA is referred to as a positive control. The target DNA has a sequence A-B. In the embodiment shown in Figure 8, the positive control is created by inserting a synthetic DNA fragment C-D, e.g. a base of 100 nucleotides, into the target sequence A-B, at scission point G. A negative control is also created, which is a synthetic DNA fragment E-F, of which the sequence is not present in the target DNA that is sought to be detected.

For example, a segment of target DNA, of about 300 bases is selected, cloned and confirmed by DNA sequencing. Based on that cloned DNA, a small fragment of about 100 bases of non-target DNA is inserted into the middle of the cloned DNA as the positive control DNA. A non-target DNA of about 200 bases is also selected, cloned and confirmed by DNA sequencing and is used as a negative control DNA. When the tests are carried out, the patient sample is added to a test tube which also contains at least the positive control, and can contain the negative control.

In a variation of the above method, the positive control may have a small fragment of DNA (about 100 bases) cut away from the target DNA.

For the purpose of the illustration in Figure 8, it is assumed that the patient has the pathogen indicator DNA, e.g. tuberculosis, which is sought to be detected. For a sample with the patient's DNA, A-B, a positive control DNA sequence A-C-D-B and a negative control DNA sequence E-F are prepared. All DNA sequences are denatured so that the patient's DNA, positive control and negative control are in single strand DNA form.

A test column 120 is prepared which has three single strand capture DNA materials. The positive control capture DNA 122 has a sequence which will bind specifically to the inserted DNA fragment C-D. The target capture DNA 123 has a sequence which will bind to the target DNA A-B on both sides of where the scission point is in the positive control DNA (point H in target DNA A-B). More specifically, point H in the target DNA sequence and the scission point G in the positive control are the same. The negative control capture DNA 124 has a sequence which will bind specifically to synthetic DNA fragment E-F. AS shown in Figure 8, positive control capture DNA 122, target capture DNA 123 and negative control capture DNA 124 are on snares 126, 127 and 128 respectively. There is a fourth snare 125 which has no DNA attached thereto. The purpose of snare 125 is for determining background "noise" in the sample. Snares 125, 126, 127 and 128 are separated longitudinally along column 120.

When performing the test for the sample, which comprises the patient's sample (target DNA, if present) and the positive and negative controls, the sample is allowed to enter column 120. It will be understood that if there is any positive control DNA A-C-D-B in the sample, it will bind with positive control capture DNA 122 with sequence C-D, leaving a sequence adjacent B, for example, free for binding to a single strand DNA probe (129). If there is any target DNA A-B in the sample, it will bind with target capture DNA 123 about scission point H, leaving a sequence adjacent B, for example, free for binding to a single strand DNA probe (129). If there is any negative control DNA E-F in the sample, it will bind with negative control capture DNA 124. Of course, if there is no target DNA A-B present, target capture DNA 123 will remain unbound. It will be understood that the positive and negative controls can be directly added into the test column separately from the patient sample.

After passing the sample through column 120, the snares are washed to remove excess single strand DNA which has not bound to any of the capture materials. After washing, a synthetic single strand DNA probe 129, which can bind with an unbound portion of the target sequence A-B , is passed through column 120. As shown in Figure 8, the sequence is close to position B. Probe 129 will attach itself to positive control DNA A-C-D-B or target DNA A-B that is present, for example at the common end sequence adjacent B, as shown in Figure 8. This method provides advantages by sharing a common sequence between the control and the target DNA for the probe binding. One advantage is that a common probe sequence can be used for both control and the target DNA. In the method illustrated by Figure 7, two different probe sequences need to be used. Furthermore, in the method demonstrated by Figure 8 the specificity and affinity of probe binding are the same between the control and the target DNA. The common reaction mechanism provides a better control for the detection process.

There is no binding of probe 129 to negative control DNA E-F because the negative control DNA E-F does not share the sequence of probe 129. Probe 129 has a detection label thereon which is used to detect the presence of the probe. If there is no detection signal associated with snare 126, then there is no positive control DNA A-C-D-B in the column. The absence of positive control signal can indicate operation errors. If there is a detection signal associated with snare 128, then the probe has bound to a material that should not have been present and the test is suspected for potential problems.

Although the negative control is a secondary control, it provides important information in addition to that obtained from a positive control. Under normal condition, the snare for negative control should never produce signals. If a signal is detected from a negative control snare, it may indicate several potential problems. For example, (1) the probe is not specific enough; (2) the column is blocked or a wash cycle is not complete; (3) wash solution is contaminated; or (4) the sample is contaminated such as an increased fluorescein concentration due to specific type of food or drug taken by the patient. Some of these could also be detected on snare 125, if it is used.

Alternatively, with the method of Figure 8 one single snare can also have thereon more than one single strand capture DNA sequences. In this case, the control and the test DNA can not share a common sequence for probe binding if they are on the same snare. The probes and labels for the control and for different target DNA are also different so that different DNA sequences can be detected on one single snare.

As indicated hereinbefore, it is possible that the signals detected from the control DNA materials and any target DNA material can be subject to some background interference caused by interaction of other materials in the sample with the snare material. The background interference can be detected from snare 125 which is independent of the control DNA materials or the target DNA material. The signals obtained from detection of the control DNA materials or the target DNA material can therefore be adjusted accordingly to take into account the interference.

The possible results are shown in Table 1 below:

**TABLE 1**

| **Test** | **Positive control** | **Target** | **Negative control** | **Result** |
|---|---|---|---|---|
| Test 1 | Positive | Positive | Negative | target DNA present |
| Test 2 | Positive | Negative | Negative | target DNA not present |
| Test 3 | Positive | Positive | Positive | Test is false positive |
| Test 4 | Negative | Negative | Negative | Test is false negative |

Thus, it can be seen that the test gives a high degree of confidence of the presence or absence of the pathogen indicator for which the test was made.

In situations where re-use is permitted, when it is desired to re-use the columns in the DNA method, the columns can be cleaned by passing a denaturing solution through the column. This causes the capture materials to revert to the single strand capture materials. Then the column is subjected to labelled probes, which should bind only with a bound target material, not capture material, and trigger chemicals and detection of any reaction. Normally, there would be no reactions detected and the column can then be washed ready for re-use. If any reaction is detected, then further treatment with denaturing solution would be used. This recycling process can be used with the DNA analysis method illustrated in Figure 8.

In another aspect, the present invention also provides a method for detection of RNA in a test sample. In previously known methods, it is necessary to isolate the RNA and convert the RNA into cDNA. This leads to loss and/or degradation of the RNA. Sometimes the loss of RNA is greater than 90%. The present method overcomes this problem and provides a greatly enhanced efficiency in the present test method in which very little, if any, RNA is lost. Additionally, as described in relation to the other embodiments of the invention, there is an increase in the reliability and accuracy of the test.

A method for detecting the presence of a RNA sequence in a test sample comprises the steps of:
(a) providing a positive control single strand DNA sequence;
(b) adding the test sample and the positive control DNA sequence to a test column wherein the column has at least two snares, one of said snares having thereon a first control single strand capture DNA sequence for binding to the positive control single strand DNA sequence; at least one of said snares having thereon a target single strand capture DNA sequence specific to the corresponding target RNA sequence in the test sample, so that the positive control DNA sequence binds with the first control single strand capture DNA sequence to form a double strand positive control DNA sequence, and the RNA sequence present in the test sample binds with the target single strand capture DNA sequence to form a double strand DNA/RNA complex;
(c) adding a wash solution to the column to remove unbound positive control DNA and target RNA;
(d) adding an enzyme to the column to destroy single strand DNA and RNA;
(e) adding a denaturing solution to separate the formed double strand control DNA sequence and double strand DNA/RNA complex, then adding a wash solution to remove denatured non-capture single strand DNA and RNA sequences, so that the single strand capture DNA sequences re-form on each snare;
(f) adding DNA probes to provide detectable labels for single strand capture DNA sequences formed in step (e);
(g) adding a wash solution to the column to remove unbound DNA probe; and
(h) detecting any signals from each snare.

The method can further include adding a substrate which reacts with the labels to give off detectable signals. There are different modes for practising the RNA detection method. In one mode, the positive control single strand DNA sequence is different from the target RNA sequence and the first control single strand capture DNA sequence is different from the target single strand capture DNA sequence. In addition, the DNA probes used in step (f) are different for the first control capture and the target capture sequences.

In another mode, the positive control single strand DNA sequence has a portion which has the same sequence to a portion of the target RNA sequence. The first control single strand capture DNA and the target single strand capture DNA have a common sequence at a portion of the capture sequences, so that a common DNA probe is used in step (f) for detection of the re-formed control and target capture sequences.

Furthermore, step (a) of the RNA detection method can further include providing a negative control single strand DNA sequence which is different from the target RNA sequence and different from the positive control DNA sequence. In this case, step (b) further includes adding the negative control DNA to the test column which also has a control snare having thereon a second control single strand capture DNA sequence. The second control capture DNA sequence partially matches the negative control DNA sequence so that the negative control DNA sequence binds with the second control capture DNA sequence to form a double strand DNA sequence which also has unbound single strand portions. Step (f) DNA probes do not match re-formed partial second control single strand capture DNA sequence formed in step (e), and no binding occurs between them. Therefore, in step (h) no signal is detected from the second control snare under normal conditions.

There are also different modes for using a negative control. In one mode, the second control single strand capture DNA sequence is different from the target capture DNA and the first control capture DNA sequences.

In an alternative mode, the first control capture DNA, the second control capture DNA and the target capture DNA have a common sequence at a portion of the capture sequences. A common DNA probe is used in step (f) for detection of the re-formed control and target capture sequences.

One mode of RNA detection process of the present invention is shown schematically in Figure 9A. Positive and negative controls are synthesized. For example, positive control DNA A-B and negative control DNA G-E are synthesized. Both of them are single strand DNA sequences. But the nucleotide sequences for the positive and negative controls are different from in the target RNA C-D.

A test column 140 is prepared which has three single strand capture DNA materials. The positive capture DNA 142 has a sequence which will bind specifically with positive control sequence A-B. The target capture DNA 143 has a sequence which will bind to the target RNA C-D if present. The negative control capture DNA 144 has a sequence E-F which will partially bind, e.g. 30-70% bind, to the negative control sequence G-E, i.e. the bound (double) strand is shorter than either of the single strand G-E sequence or the E-F sequence. As shown in Figure 9A, positive control capture DNA 142, target capture DNA 143 and negative control capture DNA 144 are on snares 146, 147 and 148 respectively. There is a fourth snare 145 which has no capture DNA attached thereto. The purpose of snare 145 is for determining background "noise" in the sample. Snares 145, 146, 147 and 148 are separated longitudinally along column 140.

When performing the test, the patient's sample (target DNA, if present) and the positive control is allowed to enter column 140. It will be understood that positive control DNA A-B will bind with positive control capture DNA 142 and if there is any target RNA C-D in the sample it will bind with target capture DNA 143. Of course, if there is no target RNA C-D present, target capture DNA 143 will remain unbound.

After passing the sample through column 140, the snares are washed, to remove excess single strand sequence which has not bound to any of the capture materials. The negative control DNA G-E is then allowed to pass through column 140. The sequence G-E will bind with target capture DNA 144. After washing, S1 nuclease is passed through the column. As S1 nuclease destroys single strand DNA, the single strand portions of negative control DNA G-E and negative control capture E-F, which are not bound to one another, will be destroyed. This will leave a small segment of double strand DNA from the negative control capture material. Clearly, if either of the other single strand capture materials (142 or 143) have not been bound, they too will be destroyed.

The column is washed again and the now double strand materials are denatured before being washed again. Subsequently, synthetic single strand DNA probes (not shown in the figure) are added to column 140 for the detection of any single strand positive control capture DNA 142 and single strand capture DNA 143 that is left in the column. The probes have detection labels thereon, which are used to detect the presence thereof. The mechanism of using single strand DNA probes for detection in the RNA analysis is the same as in the DNA analysis, which is illustrated in Figure 7.

Also similar to the DNA analysis of Figure 7, using the RNA detection method of Figure 9A one single snare can also have more than one single strand capture DNA sequences thereon. The labels are different for different single strand capture DNA sequences on one single snare so that different DNA sequences can be detected on one single snare.

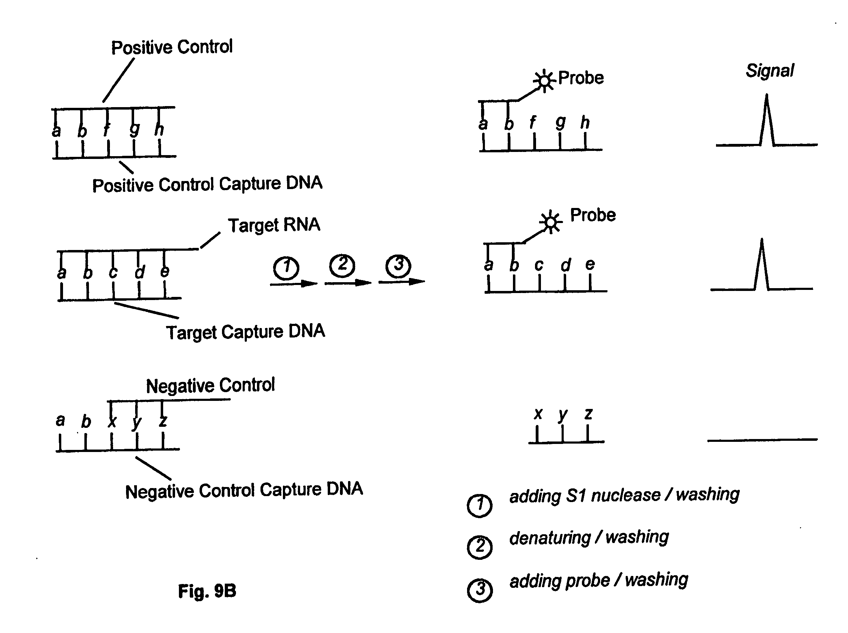

Figure 9B shows another mode of the RNA detection method. The general concept of positive control and negative control stays the same to that described above in DNA and RNA detection. The method illustrated in Figure 9B has a common single strand capture DNA portion, a-b, at one end of the three capture sequences used for capturing the positive and negative controls and the target RNA sequence, respectively. This is different from the method of Figure 9A, where the negative control capture DNA sequence does not have a portion of its sequence being the same to a portion of the positive control and the target capture sequences. Follow the same process described above for Figure 9A, after addition of S1 nuclease the common portion, a-b, of the negative control capture sequence should be destroyed by the enzyme and removed by a washing applied subsequently. Because the common portion, a-b, is an unbound single strand portion. A common probe with a label is applied after denaturing and final washing steps. The probe will bind to the common portion, a-b, of the re-formed positive control capture sequence, and the re-formed target capture sequence if the target RNA present in the sample. Signals will be detected on those snares. However, no signal should be detected on the snare thereon having the negative control sequence under normal operation conditions. Because the portion, a-b, of the negative control capture sequence would not be present then. In this case, if any signal above noise level is detected on the negative control snare, it indicates operation errors. All four possible errors discussed previously with the DNA detection method illustrated by Figure 8 could present here also. In addition, in this case, the error could also be caused by the quality and efficiency of the S1 nuclease used. Therefore, as an advantage, this method can further indicate and control the reagent and the instrument operating conditions to ensure quality of the detection.

In Figure 9B, for the convenience of illustration a common capture portion, a-b, is positioned at one end of the capture sequences. However, it is understood that a common portion can be at any position of the capture sequences. In addition, more than one common portion can present for the purpose of probe binding and subsequent detection.

A computer may be used to handle and display signals from the detectors. Additionally, a computer is preferably used to control indexing of the conveyors, activation of the introduction of reagents, control of the quantity of reagents added to the columns, and the cleaning stations if present. Use of a computer enhances the repeatability and reliability of the method. It will be understood that other control methods may be used, e.g. electromechanical methods.

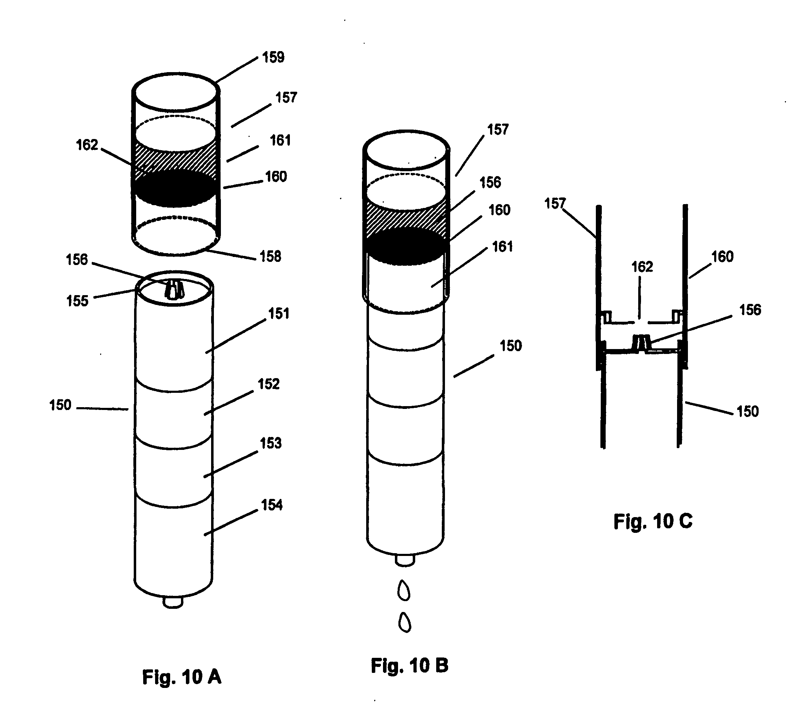

An apparatus for dispensing a sample or a reagent into the column is shown in Figure 10A to 10C. The apparatus is also called a sample dispenser which comprises a sample holder 157 and a puncturer 151. Figure 10A shows a column 150 which comprises chambers 151, 152, 153 and 154. At the top 155 of upper chamber 151 there is a delivery spout 156 which is coaxial with the longitudinal axis of column 150. The upper chamber 151 is also called a puncturer. Separate from column 150 is a sample holder 157 which has an open lower end 158 and an upper end 159 which may be closed or have means for the introduction of air or other gas. Inside sample holder 157 is an annular disk 160 with a central bore which is sealed with a film, 162, wherein the film is capable of being punctured. The sample holder 157 contains reagent 161. Preferably, the inner diameter of sample holder 157 is marginally greater than the outer diameter of chamber 151. It is understood that although Figure 10A to 10C show a cylindrical shape for both test column and sample dispenser, alternatively, other shapes such as square or rectangular shapes should also work for both column and the dispenser as long as the bore and the spout fit appropriately.

In Figure 10C, sample holder 157 is situated so that annular disk 160 is just above delivery spout 156. As sample holder 157 is lowered, the spout 156 is cause to puncture the film 162 and allow reagent 151 to flow into column 150, as will be seen in Figure 10B. In the specific example given in Figure 10A to 10C, chamber 151 has dual functions, a puncturer which punctures the film 162 to dispense the liquid sample and a column connector which provides a connection between the sample holder and the column.

The Figures of the present specification show the columns in a vertical arrangement.

In another aspect, the present invention provides a kit which comprises (a) a column for analysis of a test material; and (b) reagents for detecting the presence of the test materials. The column in the kit has at least two snares, one of the snares having thereon a first control capture material for detecting the presence of a first control material, and the other snare having thereon a capture material for detecting a test material for which detection is being sought. The column in the kit can also have at least two chambers, each chamber having a snare, one of the chambers having a first control capture material on the snare for detecting the presence of a first control material, and the other chamber having a capture material on the snare for detecting a test material for which detection is being sought. The kit can also include wash solutions for removing excess reagents from the column.

As indicated herein, the method of the present invention has wide applicability. Areas of applicability include research and diagnosis relating to cancer, auto-immune diseases, infectious diseases, haemostasis and veterinary medicine. For example, the DNA method of the present invention may be used for diagnosis of N. gonorrhoea, H. ducreyi, trepona pallidum, human papillomavirus (HPV), herpes simplex virus (HSV), molluscum contagiosum (MC), trichomonas vaginalis and the RNA method may be used for diagnosis of human immunodeficiency virus (HIV).

It will be understood that multiple tests can be carried out in a single column merely by the addition of additional snares, capture materials and detection materials. For example, the test may be used simultaneously for certain proteins, DNA sequences and RNA sequences.

The invention has been described with reference to the preferred embodiments. It should be understood, however, that the invention is not so limited, and the scope of the invention should be determined with reference to the following claims, rather than to the foregoing specification.

## Claims

1. A method for detecting the presence of a test material in a test sample comprising the steps of:
(a) introducing a test sample and a control material into the test column (40; 100), wherein the column (40; 100) has at least two snares (43-45; 106; 108; 110; 112), one of said snares having thereon a control capture material; at least one of said snares having thereon a target capture material specific to a corresponding test material in the test sample for which the detection is being sought, so that the control capture material will bind with the control material to form a bound control material; and the target capture material will bind with the corresponding test material to form a bound test material;
(b) washing the test column (40; 100) to remove materials which have not been bound to the capture materials; and
(c) detecting the presence of bound materials on each of the snares (43-45; 106; 108; 110; 112).

2. The method according to claim 1, wherein for detecting the presence of a DNA sequence in a test sample said method comprises the steps of:
(a) denaturing a test sample to form a single strand target DNA sequence (60) for which detection is being sought;
(b) introducing the test sample (60) and a first single strand control DNA sequence (68) into a test column, wherein the column has at least two snares (61; 62), one of said snares (61) having thereon a first control single strand capture DNA sequence (63); at least one of said snares (62) having thereon a target single strand capture DNA sequence (64) specific to the corresponding target DNA sequence in the test sample; and wherein the target capture DNA sequence will bind with the corresponding target DNA sequence in the test sample to form a double strand target DNA sequence, and the first control capture DNA sequence will bind with the first control DNA sequence to form a double strand control DNA sequence (69);
(c) adding a wash solution to the column to remove unbound DNA;
(d) adding an enzyme to the column to destroy single strand DNA;
(e) adding a denaturing solution (66) to separate the formed double strand DNA sequences, then adding a wash solution to remove denatured non-capture single strand sequences, so that the single strand capture DNA sequences re-form on each snare;
(f) adding DNA probes (67) to provide detectable labels for single strand capture DNA sequences formed in step (e);
(g) adding a wash solution to the column to remove unbound DNA probe;
(h) detecting any signals from each snare.

3. The method of claim 2, wherein step (b) further comprises introducing a second control single strand DNA sequence into the test column; wherein the test column also has a control snare having thereon a second oontrol single strand capture DNA sequence.

4. The method according to claim 1, wherein for detecting the presence of a DNA sequence in a test sample said method comprises the steps of:
(a) providing a positive control single strand DNA sequence;
(b) denaturing a test sample to form a single strand target DNA sequence for which detection is being sought;
(c) adding the test sample and the positive control DNA sequence to a test column (120), wherein the column (120) has at least two snares (125; 126; 127; 128), one of said snares (126) having thereon a first control single strand capture DNA sequence (122) for binding to a portion of the positive control DNA sequence; at least one of said snares (127) having thereon a target single strand capture DNA sequence (123) specific to a corresponding target DNA sequence in the test sample, so that the positive control DNA sequence binds with the first control single strand capture DNA sequence wherein the bound positive control DNA sequence has a double strand portion and a single strand portion; and the target DNA sequence present in the test sample binds with the target capture DNA sequence wherein the bound target DNA sequence has a double strand portion and a single strand portion;
(d) adding a wash solution to the column to remove unbound DNA;
(e) adding DNA probes (129) to provide detectable labels for attachment to the single strand portion of the bound positive control DNA sequence and the single strand portion of the bound target DNA sequence formed in step (c);
(f) adding a wash solution to the column to remove unbound DNA probes; and
(g) detecting any signals from each snare.

5. The method of claim 4, wherein the positive control single strand DNA sequence is prepared from a target DNA sequence for which detection is being sought, by a process selected from the group consisting of (1) inserting a control DNA fragment into the target DNA sequence at a predetermined scission point; and (2) removing a small fragment of DNA from the target DNA at a predetermined scission point.

6. The method of claim 4 or 5, wherein step (c) further includes adding a negative control DNA sequence to the test column; wherein the test column (120) also has a control snare (128) having thereon a second control single strand capture DNA sequence (124) for binding to the negative control DNA sequence, so that the negative control DNA sequence binds with the second control capture sequence to form a bound negative control DNA sequence; wherein the method further comprises preparation of a negative control single strand DNA sequence; wherein the negative control DNA sequence has a DNA sequence which is different from the target DNA sequence and different from the positive control DNA sequence.

7. The method according to claim 1, wherein for detecting the presence of an RNA sequence in a test sample said method comprises the steps of:
(a) providing a positive control single strand DNA sequence;
(b) adding a test sample and the positive control DNA sequence to a test column (140) wherein the column has at least two snares (145; 146; 147; 148), one of said snares (146) having thereon a first control single strand capture DNA sequence (142) for binding to the positive control DNA sequence; at least one of said snares (147) having thereon a target single strand capture DNA sequence (143) specific to a corresponding target RNA sequence in the test sample, so that the positive control DNA sequence binds with the first control capture DNA sequence to form a double strand positive control DNA sequence, and the RNA sequence present in the test sample binds with the target capture DNA sequence to form a double strand DNA/RNA complex;
(c) adding a wash solution to the column to remove unbound positive control DNA and target RNA;
(d) adding an enzyme to the column to destroy single strand DNA and RNA;
(e) adding a denaturing solution (66) to separate the formed double strand control DNA sequence and double strand DNA/RNA complex, then adding a wash solution to remove denatured non-capture DNA and RNA sequences, so that the single strand capture DNA sequences re-form on each snare;
(f) adding DNA probes (67) to provide detectable labels for single strand capture DNA sequences formed in step (e);
(g) adding a wash solution to the column to remove unbound DNA probes; and
(h) detecting any signals from each snare.

8. The method of claim 7, wherein the positive control DNA sequence is different from the target RNA sequence and the first control capture DNA sequence is different from the target capture DNA sequence; wherein the DNA probes used in step (f) are different for the first control capture sequence and the target capture sequence.

9. The method of claim 7, wherein the positive control DNA sequence has a portion which has the same sequence to a portion of the target RNA sequence; wherein the first control capture DNA and the target capture DNA have a common sequence at a portion of the capture sequences, so that a common DNA probe is used in step (f) for detection of the re-formed control and target capture sequences.

10. The method of any one of claims 7 to 9, wherein step (a) further includes providing a negative control single strand DNA sequence which is different from the target RNA sequence and different from the positive control DNA sequence; wherein step (b) further includes adding the negative control DNA to the test column (140) which also has a control snare (148) having thereon a second control single strand capture DNA sequence (144); wherein the second control capture DNA sequence partially matches the negative control DNA sequence so that the negative control DNA sequence binds with the second control capture DNA sequence to form a double strand DNA sequence which also has unbound single strand portions; wherein in step (f) DNA probes do not match re-formed partial second control single strand capture DNA sequence formed in step (e), and no binding occurs between the probes and the re-formed partial second control single strand capture DNA sequence, therefore, in step (h) no signal is detected from the second control snare under normal conditions.

11. The method of claim 10, wherein the first control capture DNA, the second control capture DNA and the target capture DNA have a common sequence at a portion of the capture sequences; and a common DNA probe is used in step (f) for detection of the re-formed control and target capture sequences.

12. A column for detection of a test material, wherein the column (40; 100) has at least two snares (43-45; 106; 108; 110; 112), one of said snares having thereon a first control capture material for detecting the presence of a first control material, and at least one of said snares having thereon a test capture material for detecting a test material for which detection is being sought, and wherein said snares (43-45; 106; 108; 110; 112) are spaced apart from one another.

13. The column of claim 12, wherein the column comprises at least two chambers, wherein said chambers (101; 102; 103; 104) have a connecting means to connect different chambers in order, and the chambers are connected along the longitudinal axis of the chamber through the connecting means (114; 115; 116; 117), each chamber having a snare, one of said chambers having a first control capture material on the snare for detecting the presence of a first control material, and at least one of said chambers having a test capture material on the snare for detecting the test material for which detection is being sought.

14. The column of claim 13, wherein the column further has a chamber having a second control material on the snare for detecting the presence of a second control material.

15. A kit which comprises (a) a column for analysis of a test material, wherein said column has at least two snares, one of said snares having thereon a first control capture material for detecting the presence of a first control material, and at least one of said snares having thereon a target capture material for detecting a test material for which detection is being sought, and wherein said snares are spaced apart from one another, and (b) reagents for detecting the presence of the test materials.

## Patentansprüche

1. Verfahren zum Nachweisen der Anwesenheit eines Testmaterials in einer Testprobe, umfassend die Schritte:
(a) Einbringen einer Testprobe und eines Kontrollmaterials in eine Testsäule (40; 100), wobei die Säule (40; 100) mindestens zwei Schleifen bzw. Schlingen (engl. snares) (43-45; 106; 108; 110; 112) aufweist, wobei eine der Schleifen darauf ein Kontroll-Einfang-Material aufweist, mindestens eine der Schleifen darauf ein Ziel-Einfang-Material aufweist, welches spezifisch für ein korrespondierendes Testmaterial, für das ein Nachweis gewünscht wird, in der Testprobe ist, so daß das Kontroll-Einfang-Material mit dem Kontrollmaterial binden wird, um ein gebundenes Kontrollmaterial zu bilden, und das Ziel-Einfang-Material mit dem korrespondierenden Testmaterial binden wird, um ein gebundenes Testmaterial zu bilden;
(b) Waschen der Testsäule (40; 100), um Materialien, die nicht an die Einfang-Materialien gebunden sind, zu entfernen; und
(c) Nachweisen der Anwesenheit von gebundenen Materialien auf jeder der Schleifen (43-45; 106; 108; 110; 112).

2. Verfahren nach Anspruch 1, wobei das Verfahren zum Nachweisen der Anwesenheit einer DNA-Sequenz in einer Testprobe die Schritte
(a) des Denaturierens einer Testprobe, um eine einzelsträngige Ziel-DNA-Sequenz (60), deren Nachweis gewünscht wird, zu bilden;
(b) des Einbringens der Testprobe (60) und einer ersten einzelsträngigen Kontroll-DNA-Sequenz (68) in eine Testsäule, wobei die Säule mindestens zwei Schleifen (61; 62) aufweist und eine der Schleifen (61) darauf eine erste einzelsträngige Einfang-DNA-Kontrollsequenz (63) aufweist, mindestens eine der Schleifen (62) darauf eine einzelsträngige Ziel-Einfang-DNA-Sequenz (64), welche spezifisch für die korrespondierende Ziel-DNA-Sequenz in der Testprobe ist, aufweist, und wobei die Ziel-Einfang-DNA-Sequenz mit der korrespondierenden Ziel-DNA-Sequenz in der Testprobe binden wird, um eine doppelsträngige Ziel-DNA-Sequenz zu bilden und die erste Kontroll-Einfang-DNA-Sequenz mit der ersten Kontroll-DNA-Sequenz binden wird, um eine doppelsträngige Kontroll-DNA-Sequenz (69) zu bilden;
(c) des Hinzufügens einer Waschlösung zu der Säule, um ungebundene DNA zu entfernen;
(d) des Hinzufügens eines Enzyms zu der Säule, um einzelsträngige DNA zu zerstören;
(e) des Hinzufügens einer denaturierenden Lösung (66), um die gebildeten doppelsträngigen DNA-Sequenzen zu trennen, anschließend das Hinzufügen einer Waschlösung, um denaturierte, einzelsträngige nicht-Einfang-Sequenzen zu entfernen, so daß sich die einzelsträngigen Einfang-DNA-Sequenzen auf jeder Schleife umformen bzw. erneut bilden;
(f) des Hinzufügens von DNA-Sonden (67), um nachweisbare Markierungen für einzelsträngige Einfang-DNA-Sequenzen, welche in Schritt (e) gebildet wurden, bereitzustellen;
(g) des Hinzufügens einer Waschlösung zu der Säule, um ungebundene DNA-Sonden zu entfernen;
(h) des Nachweisens jeglicher Signale von jeder Schleife
umfaßt.

3. Verfahren nach Anspruch 2, wobei Schritt (b) ferner das Einbringen einer zweiten einzelsträngigen Kontroll-DNA-Sequenz in die Testsäule umfaßt, wobei die Testsäule auch eine Kontrollschleife aufweist, die darauf eine zweite einzelsträngige Kontroll-Einfang-DNA-Sequenz aufweist.

4. Verfahren nach Anspruch 1, wobei zum Nachweisen der Anwesenheit einer DNA-Sequenz in einer Testprobe das Verfahren die Schritte
(a) des Bereitstellens einer einzelsträngigen positiven Kontroll-DNA-Sequenz;
(b) des Denaturierens einer Testprobe, um eine einzelsträngige Ziel-DNA-Sequenz, für die ein Nachweis erwünscht ist, zu bilden;
(c) des Hinzufügens der Testprobe und der positiven Kontroll-DNA-Sequenz zu einer Testsäule (120), wobei die Säule (120) mindestens zwei Schleifen (125; 126; 127; 128) aufweist und eine der Schleifen (126) darauf eine erste einzelsträngige Kontroll-Einfang-DNA-Sequenz (122) zum Binden an einen Teil der positiven Kontroll-DNA-Sequenz aufweist, mindestens eine der Schleifen (127) darauf eine einzelsträngige Ziel-Einfang-DNA-Sequenz (123), welche spezifisch für eine korrespondierende Ziel-DNA-Sequenz in der Testprobe ist, aufweist, so daß die positive Kontroll-DNA-Sequenz mit der ersten einzelsträngigen Kontroll-Einfang-DNA-Sequenz bindet, wobei die gebundene positive Kontroll-DNA-Sequenz einen doppelsträngigen Teil und einen einzelsträngigen Teil aufweist, und die in der Testprobe vorhandene Ziel-DNA-Sequenz mit der Ziel-Einfang-DNA-Sequenz bindet, wobei die gebundene Ziel-DNA-Sequenz einen doppelsträngigen Teil und einen einzelsträngigen Teil aufweist;
(d) des Hinzufügens einer Waschlösung zu der Säule, um ungebundene DNA zu entfernen;
(e) des Hinzufügens von DNA-Sonden (129), um nachweisbare Markierungen für die Anbindung bzw. Befestigung an den einzelsträngigen Teil der gebundenen positiven Kontroll-DNA-Sequenz und den einzelsträngigen Teil der gebundenen Ziel-DNA-Sequenz, welche in Schritt (c) gebildet wurde, bereitzustellen;
(f) des Hinzufügens einer Waschlösung zu der Säule, um ungebundene DNA-Sonden zu entfernen; und
(g) des Nachweisens jeglicher Signale von jeder Schleife
umfaßt.

5. Verfahren nach Anspruch 4, wobei die einzelsträngige positive Kontroll-DNA-Sequenz aus einer Ziel-DNA-Sequenz, deren Nachweis erwünscht ist, durch ein Verfahren, ausgewählt aus der Gruppe, bestehend aus (1) Einfügen eines Kontroll-DNA-Fragments in die Ziel-DNA-Sequenz an einer vorbestimmten Schnittstelle und (2) Entfernen eines kleinen DNA-Fragments von der Ziel-DNA an einer vorbestimmten Schnittstelle, hergestellt wurde.

6. Verfahren nach Anspruch 4 oder 5, wobei Schritt (c) ferner das Hinzufügen einer negativen Kontroll-DNA-Sequenz zu der Testsäule einschließt; wobei die Testsäule (120) auch eine Kontrollschleife (128) aufweist, welche darauf eine zweite einzelsträngige Kontroll-Einfang-DNA-Sequenz (124) zum Binden der negativen Kontroll-DNA-Sequenz aufweist, so daß die negative Kontroll-DNA-Sequenz mit der zweiten Kontroll-Einfang-Sequenz bindet, um eine gebundene negative Kontroll-DNA-Sequenz zu bilden, wobei das Verfahren ferner das Herstellen einer einzelsträngigen negativen Kontroll-DNA-Sequenz umfaßt, wobei die negative Kontroll-DNA-Sequenz eine DNA-Sequenz, welche von der Ziel-DNA-Sequenz verschieden ist und von der positiven Kontroll-DNA-Sequenz verschieden ist, aufweist.

7. Verfahren nach Anspruch 1, wobei zum Nachweisen der Anwesenheit einer RNA-Sequenz in einer Testprobe das Verfahren die Schritte
(a) des Bereitstellens einer einzelsträngigen positiven Kontroll-DNA-Sequenz;
(b) des Hinzufügens einer Testprobe und der positiven Kontroll-DNA-Sequenz zu einer Testsäule (140), wobei die Säule mindestens zwei Schleifen (145; 146; 147; 148) aufweist und eine der Schleifen (146) darauf eine erste einzelsträngige Kontroll-Einfang-DNA-Sequenz (142) zum Binden der positiven Kontroll-DNA-Sequenz aufweist; mindestens eine der Schleifen (147) darauf eine einzelsträngige Ziel-Einfang-DNA-Sequenz (143), welche spezifisch an die korrespondierende Ziel-RNA-Sequenz in der Testprobe bindet, aufweist, so daß die positive Kontroll-DNA-Sequenz mit der ersten Kontroll-Einfang-DNA-Sequenz bindet, um eine doppelsträngige positive Kontroll-DNA-Sequenz zu bilden, und die in der Testprobe vorhandene RNA-Sequenz mit der Ziel-Einfang-DNA-Sequenz bindet, um einen doppelsträngigen DNA/RNA-Komplex zu bilden;
(c) des Hinzufügens einer Waschlösung zu der Säule, um ungebundene positive Kontroll-DNA und Ziel-RNA zu entfernen;
(d) des Hinzufügens eines Enzyms zu der Säule, um einzelsträngige DNA und RNA zu zerstören;
(e) des Hinzufügens einer denaturierenden Lösung (66), um die gebildete doppelsträngige DNA-Sequenz und den doppelsträngigen DNA/RNA-Komplex zu trennen, anschließend des Hinzufügens einer Waschlösung, um die denaturierten nicht-eingefangenen DNA- und RNA-Sequenzen zu entfernen, so daß die einzelsträngigen Einfang-DNA-Sequenzen sich an jeder Schleife erneut bilden;
(f) des Hinzufügens von DNA-Sonden (67), um nachweisbare Markierungen für einzelsträngige Einfang-DNA-Sequenzen, die in Schritt (e) gebildet wurden, bereitzustellen;
(g) des Hinzufügens einer Waschlösung zu der Säule, um ungebundene DNA-Sonden zu entfernen; und
(h) des Nachweisens jeglicher Signale von jeder Schleife
umfaßt.

8. Verfahren nach Anspruch 7, wobei die positive Kontroll-DNA-Sequenz von der Ziel-RNA-Sequenz verschieden ist und die erste Kontroll-Einfang-DNA-Sequenz von der Ziel-Einfang-DNA-Sequenz verschieden ist, wobei die in Schritt (f) verwendeten DNA-Sonden von der ersten Kontroll-Einfang-Sequenz und der Ziel-Einfang-Sequenz verschieden sind.

9. Verfahren nach Anspruch 7, wobei die positive Kontroll-DNA-Sequenz einen Teil aufweist, welcher die gleiche Sequenz wie ein Teil der Ziel-RNA-Sequenz aufweist, wobei die erste Kontroll-Einfang-DNA und die Ziel-Einfang-DNA eine gemeinsame Sequenz an einem Teil der Einfang-Sequenzen aufweisen, so daß in Schritt (f) eine gemeinsame DNA-Sonde für den Nachweis der erneut gebildeten Kontroll- und Ziel-Einfang-Sequenzen verwendet wird.

10. Verfahren nach einem der Ansprüche 7 bis 9, wobei Schritt (a) ferner das Bereitstellen einer einzelsträngigen negativen Kontroll-DNA-Sequenz, welche von der Ziel-RNA-Sequenz verschieden ist und von der positiven Kontroll-DNA-Sequenz verschieden ist, einschließt, wobei Schritt (b) ferner das Hinzufügen der negativen Kontroll-DNA zu der Testsäule (140) einschließt, welche auch eine Kontrollschleife (148) aufweist, die darauf eine einzelsträngige zweite Kontroll-Einfang-DNA-Sequenz (144) aufweist, wobei die zweite Kontroll-Einfang-DNA-Sequenz teilweise mit der negativen Kontroll-DNA-Sequenz übereinstimmt, so daß die negative Kontroll-DNA-Sequenz mit der zweiten Kontroll-Einfang-DNA-Sequenz bindet, um eine doppelsträngige DNA-Sequenz zu bilden, welche auch ungebundene einzelsträngige Teile aufweist, wobei in Schritt (f) die DNA-Sonden nicht mit den erneut gebildeten teilweisen einzelsträngigen zweiten Kontroll-Einfang-DNA-Sequenzen, welche in Schritt (e) gebildet wurden, übereinstimmen, und keine Bindung zwischen den Sonden und den erneut gebildeten teilweisen einzelsträngigen zweiten Kontroll-Einfang-DNA-Sequenzen stattfindet, und daher in Schritt (h) unter normalen Bedingungen kein Signal von der zweiten Kontrollschleife nachgewiesen wird.

11. Verfahren nach Anspruch 10, wobei die erste Kontroll-Einfang-DNA, die zweite Kontroll-Einfang-DNA und die Ziel-Einfang-DNA eine gemeinsame Sequenz an einem Teil der Einfang-Sequenzen aufweisen und eine gemeinsame DNA-Sonde in Schritt (f) für den Nachweis der erneut gebildeten Kontroll- und Ziel-Einfang-Sequenzen verwendet wird.

12. Säule für den Nachweis von Testmaterial, wobei die Säule (40; 100) mindestens zwei Schleifen (43-45; 106; 108; 110; 112) aufweist, und wobei eine der Schleifen darauf ein erstes Kontroll-Einfang-Material zum Nachweisen der Anwesenheit eines ersten Kontrollmaterials aufweist, und mindestens eine der Schleifen darauf ein Test-Einfang-Material zum Nachweisen eines Testmaterials, für das ein Nachweis erwünscht ist, aufweist, und wobei diese Schleifen (43-45; 106; 108; 110; 112) voneinander räumlich getrennt sind.

13. Säule nach Anspruch 12, wobei die Säule mindestens zwei Kammern umfaßt, wobei die Kammern (101; 102; 103; 104) ein verbindendes Mittel zum Verbinden der verschiedenen Kammern in einer Reihenfolge aufweisen, und die Kammern entlang der Längsachse der Kammer durch die verbindenden Mittel (114; 115; 116; 117) verbunden sind, wobei jede Kammer eine Schleife aufweist, und wobei eine der Kammern ein erstes Kontroll-Einfang-Material auf der Schleife zum Nachweisen der Anwesenheit eines ersten Kontrollmaterials aufweist, und mindestens eine der Kammern ein Test-Einfang-Material auf der Schleife zum Nachweisen des Testmaterials, für das ein Nachweis erwünscht ist, aufweist.

14. Säule nach Anspruch 13, wobei die Säule ferner eine Kammer mit einem zweiten Kontrollmaterial auf der Schleife zum Nachweisen der Anwesenheit eines zweiten Kontrollmaterials aufweist.

15. Kit, welches (a) eine Säule zur Analyse eines Testmaterials, wobei die Säule mindestens zwei Schleifen aufweist, und wobei diese Schleifen darauf ein erstes Kontroll-Einfang-Material zum Nachweisen der Anwesenheit eines ersten Kontrollmaterials darauf aufweisen, und mindestens eine der Schleifen darauf ein Ziel-Einfang-Material zum Nachweisen eines Testmaterials, für das ein Nachweis erwünscht ist, aufweist, und wobei diese Schleifen räumlich getrennt voneinander angeordnet sind und (b) Reagenzien zum Nachweisen der Anwesenheit eines Testmaterials umfaßt.

## Revendications

1. Procédé pour détecter la présence d'une substance à analyser dans un échantillon à analyser comprenant les étapes consistant à :
(a) introduire un échantillon à analyser et une substance témoin dans la colonne à essai (40 ; 100), où la colonne (40 ; 100) comporte au moins deux pièges (43-45 ; 106 ; 108 ; 110 ; 112), l'un desdits pièges étant porteur d'une substance de capture témoin ; au moins l'un desdits pièges étant porteur d'une substance de capture cible spécifique d'une substance à analyser correspondante contenue dans l'échantillon à analyser que l'on cherche à détecter, de sorte que la substance de capture témoin va se lier à la substance témoin pour former une substance témoin liée ; et la substance de capture cible va se lier à la substance à analyser correspondante pour former une substance à analyser liée ;
(b) laver la colonne à essai (40 ; 100) pour éliminer les substances qui n'ont pas été liées aux substances de capture ; et
(c) détecter la présence de substances liées sur chacun des pièges (43-45 ; 106 ; 108 ; 110 ; 112).

2. Procédé selon la revendication 1, où pour détecter la présence d'une séquence d'ADN dans un échantillon à analyser ledit procédé comprend les étapes consistant à :
(a) dénaturer un échantillon à analyser pour former une séquence d'ADN cible simple brin (60) que l'on cherche à détecter ;
(b) introduire l'échantillon à analyser (60) et une première séquence d'ADN témoin simple brin (68) dans une colonne à essai, où la colonne comporte au moins deux pièges (61 ; 62), l'un desdits pièges (61) étant porteur d'une première séquence d'ADN de capture simple brin témoin (63) ; au moins l'un desdits pièges (62) étant porteur d'une séquence d'ADN de capture simple brin cible (64) spécifique de la séquence d'ADN cible correspondante contenue dans l'échantillon à analyser ; et où une séquence d'ADN de capture cible va se lier à la séquence d'ADN cible correspondante contenue dans l'échantillon à analyser pour former une séquence d'ADN cible double brin, et la première séquence d'ADN de capture témoin va se lier à la première séquence d'ADN témoin pour former une séquence d'ADN témoin double brin (69) ;
(c) ajouter une solution de lavage à la colonne pour éliminer l'ADN non lié ;
(d) ajouter une enzyme à la colonne pour détruire l'ADN simple brin ;
(e) ajouter une solution de dénaturation (66) pour séparer les séquences d'ADN double brin formées, puis ajouter une solution de lavage pour éliminer les séquences simple brin non capturées dénaturées, de sorte que les séquences d'ADN de capture simple brin se reforment sur chaque piège ;
(f) ajouter des sondes d'ADN (67) afin de fournir des marqueurs détectables pour les séquences d'ADN de capture simple brin formées à l'étape (e) ;
(g) ajouter une solution de lavage à la colonne pour éliminer la sonde d'ADN non lié ;
(h) détecter tous les signaux provenant de chaque piège.

3. Procédé selon la revendication 2, dans lequel l'étape (b) comprend, en outre, l'introduction d'une deuxième séquence d'ADN simple brin témoin dans la colonne à essai ; dans lequel la colonne à essai comporte également un piège témoin porteur d'une deuxième séquence d'ADN de capture simple brin témoin.

4. Procédé selon la revendication 1, où pour détecter la présence d'une séquence d'ADN dans un échantillon à analyser ledit procédé comprend les étapes consistant à :
(a) fournir une séquence d'ADN simple brin témoin positive ;
(b) dénaturer un échantillon à analyser pour former une séquence d'ADN cible simple brin que l'on cherche à détecter ;
(c) ajouter l'échantillon à analyser et une séquence d'ADN témoin positive dans une colonne à essai (120), où la colonne (120) comporte au moins deux pièges (125 ; 126 ; 127 ; 128), l'un desdits pièges (126) étant porteur d'une première séquence d'ADN de capture simple brin témoin (122) pour la liaison à une partie d'une séquence d'ADN témoin positive ; au moins l'un desdits pièges (127) étant porteur d'une séquence d'ADN de capture simple brin cible (123) spécifique d'une séquence d'ADN cible correspondante contenue dans l'échantillon à analyser, de sorte que la séquence d'ADN témoin positive se lie à la première séquence d'ADN de capture simple brin témoin où la séquence d'ADN témoin positive liée comporte une partie double brin et une partie simple brin ; et la séquence d'ADN cible présente dans l'échantillon à analyser se lie à la séquence d'ADN de capture cible où la séquence d'ADN cible liée comporte une partie double brin et une partie simple brin ;
(d) ajouter une solution de lavage à la colonne pour éliminer l'ADN non lié ;
(e) ajouter des sondes d'ADN (129) afin de fournir des marqueurs détectables pour identifier la liaison à la partie simple brin de la séquence d'ADN témoin positive liée et à la partie simple brin de la séquence d'ADN cible liée formées à l'étape (c) ;
(f) ajouter une solution de lavage à la colonne pour éliminer les sondes d'ADN non liés ; et
(g) détecter tous les signaux provenant de chaque piège.

5. Procédé selon la revendication 4, dans lequel la séquence d'ADN simple brin témoin positive est préparée à partir d'une séquence d'ADN cible que l'on cherche à détecter, par un procédé choisi dans le groupe consistant à (1) insérer un fragment d'ADN témoin dans la séquence d'ADN cible à un point de scission prédéterminé ; et (2) éliminer un petit fragment d'ADN de l'ADN cible à un point de scission prédéterminé.

6. Procédé selon la revendication 4 ou 5, dans lequel l'étape (c) comprend, en outre, l'ajout d'une séquence d'ADN témoin négative dans la colonne à essai ; où la colonne à essai (120) comporte également un piège témoin (128) porteur d'une deuxième séquence d'ADN de capture simple brin témoin (124) pour la liaison à la séquence d'ADN témoin négative, de sorte que la séquence d'ADN témoin négative se lie à la deuxième séquence de capture témoin pour former une séquence d'ADN témoin négative liée ; où le procédé comprend, en outre, la préparation d'une séquence d'ADN simple brin témoin négative ; où la séquence d'ADN témoin négative comporte une séquence d'ADN qui est différente de la séquence d'ADN cible et différente de la séquence d'ADN témoin positive.

7. Procédé selon la revendication 1, où pour détecter la présence d'une séquence d'ARN dans un échantillon à analyser ledit procédé comprend les étapes consistant à :
(a) fournir une séquence d'ADN simple brin témoin positive ;
(b) ajouter un échantillon à analyser et la séquence d'ADN témoin positive dans une colonne à essai (140) où la colonne comporte au moins deux pièges (145 ; 146 ; 147 ; 148), l'un desdits pièges (146) étant porteur d'une première séquence d'ADN de capture simple brin témoin (142) pour la liaison à la séquence d'ADN témoin positive ; au moins l'un desdits pièges (147) étant porteur d'une séquence d'ADN de capture simple brin cible (143) spécifique d'une séquence d'ARN cible correspondante contenue dans l'échantillon à analyser, de sorte que la séquence d'ADN témoin positive se lie à la première séquence d'ADN de capture témoin pour former une séquence d'ADN témoin positive double brin, et que la séquence d'ARN présente dans l'échantillon à analyser se lie à la séquence d'ADN de capture cible pour former un complexe ADN/ARN double brin ;
(c) ajouter une solution de lavage à la colonne pour éliminer l'ADN témoin positif et l'ARN cible non liés ;
(d) ajouter une enzyme à la colonne pour détruire l'ADN simple brin et l'ARN ;
(e) ajouter une solution de dénaturation (66) pour séparer la séquence d'ADN témoin double brin et le complexe ADN/ARN double brin formés, puis ajouter une solution de lavage pour éliminer les séquences d'ADN et d'ARN non capturées dénaturées, de sorte que les séquences d'ADN de capture simple brin se reforment sur chaque piège ;
(f) ajouter des sondes d'ADN (67) afin de fournir des marqueurs détectables pour les séquences d'ADN de capture simple brin formées à l'étape (e) ;
(g) ajouter une solution de lavage à la colonne pour éliminer les sondes d'ADN non liées ; et
(h) détecter tous les signaux provenant de chaque piège.

8. Procédé selon la revendication 7, dans lequel la séquence d'ADN témoin positive est différente de la séquence d'ARN cible et la première séquence d'ADN de capture témoin est différente de la séquence d'ADN de capture cible ; dans lequel les sondes d'ADN utilisées à l'étape (f) sont différentes pour la première séquence de capture témoin et pour la séquence de capture cible.

9. Procédé selon la revendication 7, dans lequel la séquence d'ADN témoin positive comporte une partie dont la séquence est identique à une partie de la séquence d'ARN cible ; dans lequel le premier ADN de capture témoin et l'ADN de capture cible ont une séquence commune au niveau d'une partie des séquences de capture, de sorte qu'une sonde d'ADN commune est utilisée à l'étape (f) pour la détection des séquences de capture cible et témoin reformées.

10. Procédé selon l'une quelconque des revendications 7 à 9, dans lequel l'étape (a) comprend, en outre, la fourniture d'une séquence d'ADN simple brin témoin négative qui est différente de la séquence d'ARN cible et différente de la séquence d'ADN témoin positive ; dans lequel l'étape (b) comprend, en outre, l'ajout de l'ADN témoin négatif dans la colonne à essai (140) qui comporte également un piège témoin (148) porteur d'une deuxième séquence d'ADN de capture simple brin témoin (144) ; dans lequel la deuxième séquence d'ADN de capture témoin correspond partiellement à la séquence d'ADN témoin négative de sorte que la séquence d'ADN témoin négative se lie à la deuxième séquence d'ADN de capture témoin pour former une séquence d'ADN double brin qui comporte également des parties simple brin non liées ; dans lequel à l'étape (f) les sondes d'ADN ne correspondent pas à la deuxième séquence d'ADN de capture simple brin témoin partielle reformée, formée à l'étape (e), et aucune liaison ne se produit entre les sondes et la deuxième séquence d'ADN de capture simple brin témoin partielle reformée, c'est pourquoi, à l'étape (h) aucun signal n'est détecté à partir du deuxième piège dans des conditions normales.

11. Procédé selon la revendication 10, dans lequel le premier ADN de capture témoin, le deuxième ADN de capture témoin et l'ADN de capture cible ont une séquence commune au niveau d'une partie des séquences de capture ; et une sonde d'ADN commune est utilisée à l'étape (f) pour la détection des séquences de capture cible et témoin reformées.

12. Colonne pour la détection d'une substance à analyser, où la colonne (40 ; 100) comporte au moins deux pièges (43-45 ; 106 ; 108 ; 110 ; 112), l'un desdits pièges étant porteur d'une première substance de capture témoin afin de détecter la présence d'une première substance témoin, et au moins l'un desdits pièges étant porteur d'une substance de capture à analyser afin de détecter une substance à analyser que l'on cherche à détecter, et où lesdits pièges (43-45 ; 106 ; 108 ; 110 ; 112) sont espacés les uns des autres.

13. Colonne selon la revendication 12, où la colonne comprend au moins deux chambres, où lesdites chambres (101 ; 102 ; 103 ; 104) comportent des moyens de connexion afin de relier les différentes chambres selon un ordre établi, et les chambres sont reliées le long d'un axe longitudinal de la chambre par les moyens de connexion (114 ; 115 ; 116 ; 117), chaque chambre ayant un piège, l'une desdites chambres ayant une première substance de capture témoin sur le piège afin de détecter la présence d'une première substance témoin, et au moins l'une desdites chambres ayant une substance de capture à analyser sur le piège afin de détecter la substance à analyser que l'on cherche à détecter.

14. Colonne selon la revendication 13, où la colonne comprend, en outre, une chambre comportant une deuxième substance témoin sur le piège afin de détecter la présence d'une deuxième substance témoin.

15. Trousse comprenant (a) une colonne pour analyser une substance à analyser, dans laquelle ladite colonne comporte au moins deux pièges, l'un desdits pièges étant porteur d'une première substance de capture témoin afin de détecter la présence d'une première substance témoin, et au moins l'un desdits pièges étant porteur d'une substance de capture cible afin de détecter une substance à analyser que l'on cherche à détecter, et dans laquelle lesdits pièges sont espacés les uns des autres, et (b) des réactifs pour détecter la présence des substances à analyser.
